(19) Europäisches Patentamt
European Patent Office
Office européen des brevets

(11) EP 3 708 659 A1

(12) EUROPEAN PATENT APPLICATION

(43) Date of publication:
**16.09.2020 Bulletin 2020/38**

(21) Application number: **20168557.5**

(22) Date of filing: **22.09.2017**

(51) Int Cl.:
***C12N 9/10*** *(2006.01)* ***C12P 13/10*** *(2006.01)*
***C12P 7/64*** *(2006.01)* ***C11D 1/52*** *(2006.01)*
***C11D 1/64*** *(2006.01)* ***C12P 13/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.09.2016 GB 201616230**

(62) Document number(s) of the earlier application(s) in accordance with Art. 76 EPC:
**17784696.1 / 3 516 065**

(71) Applicant: **University Of The Western Cape**
**7530 Cape Town (ZA)**

(72) Inventor: **WILLIAMS, Wesley Trevor**
**7780 Cape Town (ZA)**

(74) Representative: **Appleyard Lees IP LLP**
**15 Clare Road**
**Halifax HX1 2HY (GB)**

Remarks:
This application was filed on 07-04-2020 as a divisional application to the application mentioned under INID code 62.

# (54) HETEROLOGOUS EXPRESSION OF ORNITHINE ACYL-ACP N-ACYLTRANSFERASES AND THEIR USES

(57) The present invention relates to a method for heterologously expressing an ornithine acyl-ACP N-acyltransferase in a microorganism, wherein the expression of the ornithine acyl-ACP N-acyltransferase protein catalyses the synthesis of lyso-ornithine lipid in the microorganism. The invention further provides for the production of an ornithine lipid in instances where the microorganism endogenously expresses lyso-ornithine dependent acyl-ACP O-acyltransferase. The invention also provides for recombinant microorganisms capable of producing lyso-ornithine and ornithine lipids which have application as biosurfactants, specifically emulsification activity.

EP 3 708 659 A1

**Description**

## BACKGROUND OF THE INVENTION

The present invention relates to a method for heterologously expressing an ornithine acyl-ACP N-acyltransferase in a microorganism, wherein the expression of the ornithine acyl-ACP N-acyltransferase protein catalyses the synthesis of lyso-ornithine lipid in the microorganism. The invention further provides for the production of an ornithine lipid in instances where the microorganism endogenously expresses lyso-ornithine dependent acyl-ACP O-acyltransferase. The invention also provides for recombinant microorganisms capable of producing lyso-ornithine and ornithine lipids which have application as biosurfactants and have specific emulsification activity.

Biosurfactants are biodegradable and environmentally compatible substitutes for petrochemically produced surfactants. Their mild surfactant properties and wide operational capacity has identified them as suitable candidates to replace surfactants in certain applications whose economics suit their low production volumes. The main biosurfactant molecules mooted for industrial applications are sophorolipids, rhamnolipids and mannosylerythritol lipids, but the lack of diversity of biosurfactant structures is one of the problems requiring remedies if these molecules are to find widespread surfactant industry acceptance.

Identification of biosurfactants in the past relied completely on the culture based approach (Jackson *et al.* 2015b). A typical biosurfactant screening study would use a minimal media supplemented with a plant or mineral oil as a carbon source. Only microorganisms that are able to mobilise the hydrophobic carbon substrates would be selected (Bodour *et al.* 2003; Cai *et al.* 2014; Jackson *et al.* 2015b). The physical effect of the produced biosurfactant on the culture supernatant would then be measured by several well established methods (Walter *et al.* 2010). While, the culture based approach has been successful at the identification of many useful secondary metabolites, it favours only microorganisms capable of being cultured in the media (Trindade *et al.* 2015). In order to access the majority of the diversity that are not culturable, the functional metagenomic approach is useful, where the total metagenome is cloned and expressed in a heterologous host, and is then screened. This study is the first to use the functional metagenomic approach for the biodiscovery of biosurfactants (Kennedy *et al.* 2008; Jackson *et al.* 2015b), in order to access many potential biosurfactant molecules that might lie undiscovered in the uncultured microbial diversity of many environments.

Central to the assembly of biosurfactant structures *in vivo* is the transfer of the fatty acid tail to the polar head group by acyltransferase. Acyltransferases have a magnitude of other functions not involving biosurfactant synthesis, therefore sequence based search strategies would be insufficient for biodiscovery of new biosurfactant structures. Furthermore, certain biosurfactants, such as rhamnolipids possess relatively conserved gene sequences for their transferase gene sequences leading to the re-isolation of already known gene sequences or producers. The functional screening method remains the best hope for identifying novel, rare or previously unconsidered biosurfactant genes.

However, the functional metagenomic approach too has limitations for biosurfactant discovery. By combining a culture dependant and independent approach in parallel these methods could work synergistically to identify biosurfactant molecules from both culturable and as-yet-uncultured microorganisms.

## SUMMARY OF THE INVENTION

According to a first aspect of the invention there is provided for a method for producing an ornithine-containing lipid in a recombinant microorganism, comprising heterologously expressing an ornithine acyl-ACP N-acyltransferase in the microorganism, wherein the expression of the ornithine acyl-ACP N-acyltransferase catalyses the synthesis of lyso-ornithine lipid in the microorganism. The lyso-ornithine lipid may comprise the structure set out in formula (I):

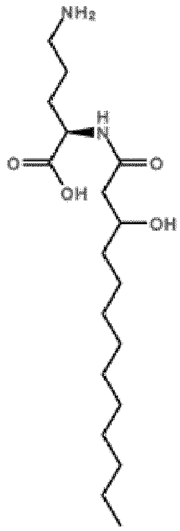

(I)

In a first embodiment of the invention the ornithine acyl-ACP N-acyltransferase has an amino acid sequence substantially identical to SEQ ID NO:2 and is heterologously expressed by an *olsB*-like gene having at least 90% sequence identity to SEQ ID NO:1.

In a second embodiment of the invention the microorganism is a gram positive or gram negative bacterium. Preferably the microorganism is selected from *Pseudomonas putida* or *Escherichia coli.*

In a further embodiment of the invention the microorganism endogenously expresses a lyso-ornithine dependent acyl-ACP O-acyltransferase enzyme. It will be appreciated that the lyso-ornithine dependent acyl-ACP O-acyltransferase enzyme may be expressed from an *olsA*-gene present in the microorganism.

In yet another embodiment of the invention the lyso-ornithine dependent acyl-ACP O-acyltransferase catalyses the synthesis of an ornithine lipid. The ornithine lipid may comprise the structure of formula (II):

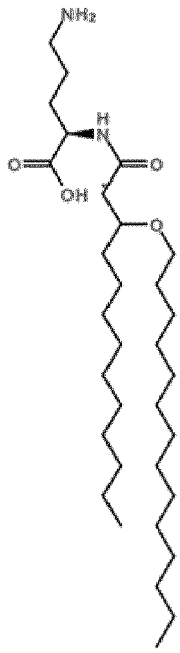

(II)

According to a second aspect of the invention there is provided for a recombinant microorganism capable of heterologously expressing an ornithine acyl-ACP N-acyltransferase which catalyses the synthesis of an ornithine-containing lipid, wherein the ornithine-containing lipid is lyso-ornithine lipid.

In one embodiment of the invention the ornithine acyl-ACP N-acyltransferase has an amino acid sequence substantially identical to SEQ ID NO:2 and is heterologously expressed by an *olsB*-like gene having at least 90% sequence identity with SEQ ID NO:1.

In another embodiment of the invention the recombinant microorganism is selected from *Pseudomonas putida* or *Escherichia coli.*

In yet another embodiment of the invention the recombinant microorganism endogenously expresses lyso-ornithine dependent acyl-ACP O-acyltransferase which catalyses the synthesis of ornithine lipid from the lyso-ornithine lipid. Preferably the lyso-ornithine dependent acyl-ACP O-acyltransferase is expressed from an *olsA* or *olsA*-like gene.

In a further embodiment of the invention the ornithine-containing lipid is ornithine lipid.

According to a third aspect of the invention there is provided for an ornithine-containing lipid produced by the methods of the first aspect of the invention or a lipid produced by the recombinant organism of the second aspect of the invention.

In one embodiment the lipid is an ornithine-containing lipid selected from lyso-ornithine lipid or ornithine lipid.

In a second embodiment of the invention the ornithine-containing lipid may be used as a biosurfactant. The biosurfactant may be used as an emulsifier, and the ornithine-containing lipid preferably has an emulsification capacity after 24 hours ($EC_{24}$) of at least about 62,5%, preferably at least about 65%, more preferably at least about 70%, when emulsifying in paraffin.

According to a fourth aspect of the invention, there is provided for a biosurfactant, comprising an ornithine-containing lipid or ornithine lipid produced by the methods of the first aspect of the invention or a lipid produced by the recombinant organism of the second aspect of the invention. The biosurfactant preferably has emulsification activity, more preferably an emulsification capacity after 24 hours ($EC_{24}$) of at least about 62,5%, preferably at least about 65%, more preferably at least about 70%, when emulsifying in paraffin.

## BRIEF DESCRIPTION OF THE FIGURES

Non-limiting embodiments of the invention will now be described by way of example only and with reference to the following figures:

**Figure 1:** A 0.8% agarose gel analysing representative fosmids extracted from the metagenomics clone library (LSSpCCFOS2) to determine the average insert size of the library.

**Figure 2:** A photograph of a *Pseudomonas* Selective Agar plate containing the *P. putida* E54F3 clone identified with biosurfactant activity by the droplet paraffin spray method. The halo around the colonies is clearly visible (arrows)

**Figure 3:** A photograph of the emulsification of paraffin and LB culture supernatant of *P. putida*-E54F3. The emulsion had an $EC_{24}$ of 70%.

**Figure 4:** Photographs of the emulsions formed by (A) *P. putida* MBD1, *P. putida*-E54F3 and *E.* co*li*-E54F3 taken 24 hours after emulsification. (B) Tilted test tubes of emulsifications of LB supernatants from *P. putida* and *P. putida*+E54F3 demonstrate the comparative stability of the emulsions formed.

**Figure 5:** Multiple sequence alignment of representative sequences of the COG3176; biochemically confirmed OlsB protein sequences of *S. meliloti* and *P. aeruginosa* PA01; OlsB sequences from known OL producers *B. xenovorans*, *B. psuedomallei* and *M. loti*; and the closely related sequences of ORF32 annotated as haemolysins (Table 1) of *P. lavamentivorans* and *Methylobacterium* sp. Leaf113.

**Figure 6**: The biosynthetic pathway of ornithine lipid requires two-steps. The first step is catalysed by OlsB, the ornithine is N-acylated to form lyso-ornithine lipid (Gao et al. 2004) after which OlsA O-acylates the lyso-ornithine lipid to form ornithine lipid (Weissenmayer et al. 2002).

**Figure 7:** Shake flasks of *E. coli* BL21-ORF32 containing increasing concentrations of ornithine (A). The emulsification of culture supernatant and an equal volume of paraffin after 24 hours. An increase of foam and emulsification capacity with increasing concentrations of ornithine was observed.

**Figure 8:** A microwell plate assay for surface tension reduction. The first (top) row is the controls of 20 % SDS, LB and $dH_2O$. The second row is culture supernatants of *E. coli* pET21a-ORF32 with and without IPTG and 10 mM ornithine in various combinations, which shows all the cultures positive for surface tension reduction. The third row is culture supernatants of *E. coli* BL21 with and without IPTG and 10 mM ornithine. No surface tension reduction is visible.

**Figure 9:** Emulsion stability after 24 hours for *E. coli* BL21 culture supernatants and *E. coli* pET21a-ORF32 culture supernatants. The cultures containing +IPTG and +Orn was supplemented with 1 mM IPTG and 10 mM ornithine. The emulsions formed with paraffin after 24 hours.

**Figure 10:** The emulsions of *E. coli* BL21-ORF32 containing 0 mM and 10 mM ornithine were disrupted by brief vortex. The emulsion of the 0 mM ornithine culture collapse while the 10 mM ornithine culture continued to form an emulsion with paraffin.

**Figure 11:** Water in oil emulsions of culture supernatants and paraffin. A) Emulsion of *E. coli* BL21 without ORF32. B) Emulsion of *E. coli* BL21-ORF32 culture without IPTG and ornithine supplementation. C) Emulsion of *E. coli* BL21-ORF32 culture supernatant with 1 mM IPTG and 10 mM ornithine. The photographs were taken under 10 x magnification.

**Figure 12:** The emulsification of paraffin by *P. putida* MBD1-E54F3 and the knock out ORF32 mutant *P. putida* MBD1-E54F3_D1E5. Both cultures show slight emulsification activity under high phosphate conditions (A and B). Under low phosphate conditions *P. putida* MBD1-E54F3's $EC_{24}$ was 70% (C), while *P. putida* MBD1-E54F3_D1 E5 disappears almost entirely.

**Figure 13:** A photo of the *P. putida* MBD1-E54F3 BM2 culture supernatant and paraffin emulsion held horizontally to illustrate the stability of the emulsion.

**Figure 14:** A TLC stained by 0.1% ninhydrin. The total lipid was extracted from *P. putida* MBD1-E54F3 and *P. putida* MBD1-E54F3_D1E5. The bacteria were grown under two phosphate conditions, high phosphate (1600 μM $PO_4$) and low phosphate (400 μM).

**Figure 15:** Comparison of putative ornithine lipid production of overnight cultures of *P. putida* MBD1-E54F3 and *P. putida* MBD1 cultured in BM2 media supplemented with 400 μM phosphate and chemically synthesised lyso-ornithine lipid (LOL). Culturing using 400 μM phosphate and extraction of *P. putida* MBD1-E54F3 (lanes 2 to 4) and *P. putida* MBD1 (lanes 5 to 7) was conducted in triplicate. Synthetic LOL (lane 9) was used as a control to identify the presence of any LOL produced by *P. putida* MBD1-E54F3 or *P. putida* MBD1. Where *P. putida* MBD1-E54F3 consistently produced the putative OL spot, *P. putida* MBD1 produced a weaker and inconsistent spot representing OL. A single TLC photo was cropped and merged using PowerPoint.

**Figure 16:** *P. putida* MBD1-E54F3 was cultured in BM2 media (400 μM $PO_4$) and total lipid extracts were separated by TLC. The suspected ornithine lipid band was scraped and extracted. The sample was then subjected to mass spectrometry.

**Figure 17:** The structures determined for signals 652.73 (623 m/z), 651.72 (649 m/z) and 665 (663 m/z) by negative mass spray tandem MS show the different chain lengths for the amide fatty acid.

**Figure 18:** The negative ion electrospray tandem MS of the major signal 651 m/z in *P. aeruginosa* PA01 determined by Lewenza *et al.* which correspond to our most prominent signal. The diagram was taken from (Lewenza *et al.* 2011).

**Figure 19:** Nucleotide sequence of ORF32 (SEQ ID NO:1).

**Figure 20:** Predicted amino acid sequence of ORF32 (SEQ ID NO:2).

## SEQUENCE LISTING

The nucleic acid and amino acid sequences listed in the accompanying sequence listing are shown using standard letter abbreviations for nucleotide bases, and the standard three letter abbreviations for amino acids. It will be understood by those of skill in the art that only one strand of each nucleic acid sequence is shown, but that the complementary strand is included by any reference to the displayed strand. The accompanying sequence listing is hereby incorporated by reference in its entirety.

## DETAILED DESCRIPTION OF THE INVENTION

The present invention will now be described more fully hereinafter with reference to the accompanying drawings, in which some, but not all embodiments of the invention are shown.

The invention as described should not be limited to the specific embodiments disclosed and modifications and other embodiments are intended to be included within the scope of the invention. Although specific terms are employed herein, they are used in a generic and descriptive sense only and not for purposes of limitation.

As used throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having" and "including" and variations thereof used herein, are meant to encompass the items listed thereafter and equivalents thereof as well as additional items.

A "protein," "peptide" or "polypeptide" is any chain of two or more amino acids, including naturally occurring or non-naturally occurring amino acids or amino acid analogues, irrespective of post-translational modification (e.g., glycosylation or phosphorylation).

The terms "nucleic acid", "nucleic acid molecule" and "polynucleotide" are used herein interchangeably and encompass both ribonucelotides (RNA) and deoxyribonucleotides (DNA), including cDNA, genomic DNA, and synthetic DNA. The nucleic acid may be double-stranded or single-stranded. Where the nucleic acid is single-stranded, the nucleic acid may be the sense strand or the antisense strand. A nucleic acid molecule may be any chain of two or more covalently bonded nucleotides, including naturally occurring or non-naturally occurring nucleotides, or nucleotide analogs or derivatives. By "RNA" is meant a sequence of two or more covalently bonded, naturally occurring or modified ribonucleotides. The term "DNA" refers to a sequence of two or more covalently bonded, naturally occurring or modified deoxyribonucleotides.

The term "isolated", is used herein and means having been removed from its natural environment.

The term "purified", relates to the isolation of a molecule or compound in a form that is substantially free of contamination or contaminants. Contaminants are normally associated with the molecule or compound in a natural environment, purified thus means having an increase in purity as a result of being separated from the other components of an original composition. The term "purified nucleic acid" describes a nucleic acid sequence that has been separated from other compounds including, but not limited to polypeptides, lipids and carbohydrates which it is ordinarily associated with in its natural state.

The term "complementary" refers to two nucleic acids molecules, e.g., DNA or RNA, which are capable of forming Watson-Crick base pairs to produce a region of double-strandedness between the two nucleic acid molecules. It will be appreciated by those of skill in the art that each nucleotide in a nucleic acid molecule need not form a matched Watson-Crick base pair with a nucleotide in an opposing complementary strand to form a duplex. One nucleic acid molecule is thus "complementary" to a second nucleic acid molecule if it hybridizes, under conditions of high stringency, with the second nucleic acid molecule. A nucleic acid molecule according to the invention includes both complementary molecules.

As used herein a "substantially identical" sequence is an amino acid or nucleotide sequence that differs from a reference sequence only by one or more conservative substitutions, or by one or more non-conservative substitutions, deletions, or insertions located at positions of the sequence that do not destroy or substantially reduce the activity of one or more of the expressed polypeptides or of the polypeptides encoded by the nucleic acid molecules. Alignment for purposes of determining percent sequence identity can be achieved in various ways that are within the knowledge of those with skill in the art. These include using, for instance, computer software such as ALIGN, Megalign (DNASTAR), CLUSTALW or BLAST software. Those skilled in the art can readily determine appropriate parameters for measuring alignment, including any algorithms needed to achieve maximal alignment over the full length of the sequences being compared. In one embodiment of the invention there is provided for a polypeptide or polynucleotide sequence that has at least about 80% sequence identity, at least about 90% sequence identity, or even greater sequence identity, such as about 95%, about 96%, about 97%, about 98% or about 99% sequence identity to the sequences described herein.

Alternatively, or additionally, two nucleic acid sequences may be "substantially identical" if they hybridize under high stringency conditions. The "stringency" of a hybridisation reaction is readily determinable by one of ordinary skill in the art, and generally is an empirical calculation which depends upon probe length, washing temperature, and salt concentration. In general, longer probes required higher temperatures for proper annealing, while shorter probes require lower temperatures. Hybridisation generally depends on the ability of denatured DNA to re-anneal when complementary strands are present in an environment below their melting temperature. A typical example of such "stringent" hybridisation conditions would be hybridisation carried out for 18 hours at 65°C with gentle shaking, a first wash for 12 min at 65°C in Wash Buffer A (0.5% SDS; 2XSSC), and a second wash for 10 min at 65°C in Wash Buffer B (0.1% SDS; 0.5% SSC).

Those skilled in the art will appreciate that polypeptides, peptides or peptide analogues can be synthesised using standard chemical techniques, for instance, by automated synthesis using solution or solid phase synthesis methodology. Automated peptide synthesisers are commercially available and use techniques known in the art. Polypeptides, peptides and peptide analogues can also be prepared from their corresponding nucleic acid molecules using recombinant DNA technology.

As used herein, the term "gene" refers to a nucleic acid that encodes a functional product, for instance an RNA, polypeptide or protein. A gene may include regulatory sequences upstream or downstream of the sequence encoding the functional product.

As used herein, the term "coding sequence" refers to a nucleic acid sequence that encodes a specific amino acid sequence. On the other hand a "regulatory sequence" refers to a nucleotide sequence located either upstream, downstream or within a coding sequence. Generally regulatory sequences influence the transcription, RNA processing or stability, or translation of an associated coding sequence. Regulatory sequences include but are not limited to: effector binding sites, enhancers, introns, polyadenylation recognition sequences, promoters, RNA processing sites, stem-loop structures, translation leader sequences;.

In some embodiments, the genes used in the method of the invention may be operably linked to other sequences. By "operably linked" is meant that the nucleic acid molecules encoding the ornithine acyl-ACP N-acyl transferase polypeptides of the invention and regulatory sequences are connected in such a way as to permit expression of the proteins when the appropriate molecules are bound to the regulatory sequences. Such operably linked sequences may be contained in vectors or expression constructs which can be transformed or transfected into host cells for expression. It will be appreciated that any vector or vectors can be used for the purposes of expressing ornithine acyl-ACP N-acyl transferase of the invention.

The term "promoter" refers to a DNA sequence that is capable of controlling the expression of a nucleic acid

coding sequence or functional RNA. A promoter may be based entirely on a native gene or it may be comprised of different elements from different promoters found in nature. Different promoters are capable of directing the expression of a gene in different cell types, or at different stages of development, or in response to different environmental or physiological conditions. A "constitutive promoter" is a promoter that direct the expression of a gene of interest in most host cell types most of the time.

The term "recombinant" means that something has been recombined. When used with reference to a nucleic acid construct the term refers to a molecule that comprises nucleic acid sequences that are joined together or produced by means of molecular biological techniques. The term "recombinant" when used in reference to a protein or a polypeptide refers to a protein or polypeptide molecule which is expressed from a recombinant nucleic acid construct created by means of molecular biological techniques. Recombinant nucleic acid constructs may include a nucleotide sequence which is ligated to, or is manipulated to become ligated to, a nucleic acid sequence to which it is not ligated in nature, or to which it is ligated at a different location in nature. Accordingly, a recombinant nucleic acid construct indicates that the nucleic acid molecule has been manipulated using genetic engineering, i.e. by human intervention. Recombinant nucleic acid constructs may be introduced into a host cell by transformation. Such recombinant nucleic acid constructs may include sequences derived from the same host cell species or from different host cell species.

As used herein, the term "chimeric", means that a sequence comprises of sequences that have been "recombined". By way of example sequences are recombined and are not found together in nature. The term "recombine" or "recombination" refers to any method of joining two or more polynucleotides. The term includes end to end joining, and insertion of one sequence into another. The term is intended to include physical joining techniques, for instance, sticky-end ligation and blunt-end ligation. Sequences may also be artificially synthesized to contain a recombined sequence. The term may also encompass the integration of one sequence into a second sequence by way of, for example, homologous recombination

The term "vector" refers to a means by which polynucleotides or gene sequences can be introduced into a cell. There are various types of vectors known in the art including plasmids, viruses, bacteriophages and cosmids. Generally polynucleotides or gene sequences are introduced into a vector by means of a cassette. The term "cassette" refers to a polynucleotide or gene sequence that is expressed from a vector, for example, the polynucleotide or gene sequences encoding the acyl transferase polypeptides of the invention. A cassette generally comprises a gene sequence inserted into a vector, which in some embodiments, provides regulatory sequences for expressing the polynucleotide or gene sequences. In other embodiments, the vector provides the regulatory sequences for the expression of the acyl transferase polypeptides. In further embodiments, the vector provides some regulatory sequences and the nucleotide or gene sequence provides other regulatory sequences. "Regulatory sequences" include but are not limited to promoters, transcription termination sequences, enhancers, splice acceptors, donor sequences, introns, ribosome binding sequences, poly(A) addition sequences, and/or origins of replication.

The following paragraphs describe embodiments of the present invention.

Paragraph 1. A method for producing an ornithine-containing lipid in a microorganism, the method comprising: heterologously expressing an ornithine acyl-ACP N-acyltransferase in the microorganism, wherein the expression of the ornithine acyl-ACP N-acyltransferase catalyses the synthesis of lyso-ornithine lipid.

Paragraph 2. The method of paragraph 1, wherein the ornithine acyl-ACP N-acyltransferase has an amino acid sequence substantially identical to SEQ ID NO:2.

Paragraph 3. The method of paragraph 1 or paragraph 2, wherein the ornithine acyl-ACP N-acyltransferase is heterologously expressed by an *olsB*-like gene having at least 90% sequence identity with SEQ ID NO:1.

Paragraph 4. The method of any one of paragraphs 1 to 3, wherein the microorganism is selected from *Pseudomonas putida* or *Escherichia coli.*

Paragraph 5. The method of any one of paragraphs 1 to 4, wherein the microorganism endogenously expresses lyso-ornithine dependent acyl-ACP O-acyltransferase.

Paragraph 6. The method of paragraph 5, wherein the lyso-ornithine dependent acyl-ACP O-acyltransferase is expressed by an *olsA*-gene present in the microorganism.

Paragraph 7. The method of paragraph 5 or paragraph 6, wherein the lyso-ornithine dependent acyl-ACP O-acyltransferase catalyses the synthesis of ornithine lipid.

Paragraph 8. A recombinant microorganism capable of heterologously expressing an ornithine acyl-ACP N-acyltransferase which catalyses the synthesis of an ornithine-containing lipid, wherein the ornithine-containing lipid is lyso-ornithine lipid.

Paragraph 9. The recombinant microorganism of paragraph 8, wherein the ornithine acyl-ACP N-acyltransferase has an amino acid sequence substantially identical to SEQ ID NO:2.

Paragraph 10. The recombinant microorganism of paragraph 8 or paragraph 9, wherein the ornithine acyl-ACP N-acyltransferase is heterologously expressed by an *olsB*-like gene having at least 90% sequence identity with SEQ ID NO:1.

Paragraph 11. The recombinant microorganism of any one of paragraphs 8 to 10, wherein the recombinant microorganism is selected from recombinant *Pseudomonas putida* or *Escherichia coli.*

Paragraph 12. The recombinant microorganism of any one of paragraphs 8 to 11, wherein the recombinant microorganism endogenously expresses lyso-ornithine dependent acyl-ACP O-acyltransferase.

Paragraph 13. The recombinant microorganism of paragraph 12, wherein the lyso-ornithine dependent acyl-ACP O-acyltransferase is expressed by an *olsA*-gene present in the microorganism.

Paragraph 14. The recombinant microorganism of paragraph 12 or paragraph 13, wherein the ornithine-containing lipid is ornithine lipid.

Paragraph 15. An ornithine-containing lipid produced by the method of any one of paragraphs 1 to 7 or the recombinant organism of any one of paragraph 8 to 14.

Paragraph 16. The ornithine-containing lipid of paragraph 15, wherein the ornithine-containing lipid is lyso-ornithine lipid or ornithine lipid.

Paragraph 17. The ornithine-containing lipid of clai paragraph 16, for use as a biosurfactant.

Paragraph 18. The ornithine-containing lipid of paragraph 17, wherein the biosurfactant has an emulsification capacity after 24 hours ($EC_{24}$) of at least about 65% when emulsifying in paraffin.

Paragraph 19. A biosurfactant, comprising an ornithine-containing lipid produced by the method of any one of paragraphs 1 to 7 or the recombinant organism of any one of paragraphs 8 to 14.

Paragraph 20. The biosurfactant of paragraph 12, having an emulsification capacity after 24 hours ($EC_{24}$) of at least about 65% when emulsifying in paraffin.

The following examples are offered by way of illustration and not by way of limitation.

## EXAMPLE 1

*Sampling*

Lake sediment soil samples with superficial boat diesel contamination were obtained from Bufflespruit Lake, Bela Bela, Northwest Province, South Africa, November 2013 (S 24 50 49.1, E 28 14 31.4).

The Bufflespruit Dam contained a section of soil with apparent diesel contamination due to the presence of recreation motor boats. Hydrocarbon contamination is associated with the selection of biosurfactant producing microorganisms, therefore could have served in enriching for biosurfactant-producing microorganisms.

*Direct DNA extraction*

Soil metagenomic DNA was extracted using the 'Zhou method' using SDS and CTAB to directly lyse bacterial cells within the soil, followed by organic extraction with chloroform isoamyl alcohol of the DNA, which was then precipitated by isopropanol (Zhou ***et al.*** 1996). Soil (5 g) was mixed with 13.5 ml Extraction Buffer and 100 μl proteinase K (100 mg/ml) in 15 ml Oakridge centrifuge tubes. The sample was incubated at 37°C for 30 minutes at 225 rpm. After incubation 1.5 ml 20% SDS was added and incubated in a water bath at 65°C for 2 hours with gently inversion every 15 to 20 minutes. The supernatant was collected by centrifugation at 4000 x g for 10 minutes at room temperature and transferred to a 50 ml Falcon tube. Another 4.5 ml Extraction Buffer was added to the soil sample and mixed by vortex for 10 seconds followed by incubation at 65°C for 10 minutes. The supernatant was collected as before. A total of three cycles of extractions was performed and pooled for organic extraction. An equal volume of chloroform: isoamyl alcohol (24:1, v/v) (CI) was added to the supernatant and gently extracted by shaking on a belly dancer for 10 minutes. The sample was centrifuged at 6000 x g for 20 minutes and the upper aqueous phase was recovered by aspiration using wide-bore pipette tips. Isopropanol (0.6 volumes) was added to the aqueous phase and incubated at room temperature for 1 hour to precipitate the DNA. The precipitated DNA was pelleted by centrifugation at 16 000 x g for 20 minutes at room temperature then washed with 5 ml cold 70% ethanol and centrifuged at 16 000 x g for 10 minutes at 4°C. The pellet was suspended in sterile $dH_2O$ to a final volume of 500 μl, a 100 μl for every 1 g of soil used for extraction.

*Size Selection of HMW DNA*

Impurities that interfere with the library production procedures were removed by sodium chloride/formamide wash solution and agarose gel electrophoresis (Liles ***et al***. 2008). The crude DNA extract (500 μl) was mixed with 500 μl molten 2% TAE agarose, drawn into a 1 ml syringe to form a 1% agarose plug. After solidification, the plug was washed in 50 ml formamide solution (80% formamide, 1.3 M NaCl) overnight. The plug was transferred 50 ml Falcon tube containing Tris-EDTA (10mM Tris-HCl, 1 mM EDTA) (TE) buffer and washed multiple times to remove the formamide solution.

High molecular weight DNA was retrieved from the plug by electrophoresis using a well suitably sized for the agarose plug (±1 cm in length) in 1% low melting point (LMP) TAE agarose and electrophoresis at 30 V for 16 hours. The HMW DNA above the 23.13 kb mark of λ-DNA digested by *HindIII* was excised using a sterile scalpel without exposing the DNA to any UV light then melted at 70°C for 10 minutes. The liquid agarose was digested using 1 U per gram agarase (Fermentas) in a 42°C water bath for 30 minutes. The digested agarose was divided into 500 μl aliquots, transferred to 1.5 ml microcentrifuge tubes and incubated on ice for 5 minutes then centrifuged at maximum speed in a bench top centrifuge for 20 minutes at 4°C. The upper 95% of the supernatant was recovered while the gelatinous pellet was avoided. A tenth of a volume 3 M sodium acetate (pH 7.0) and 2.5 volumes of absolute ethanol was added to the supernatant. The DNA was precipitated at room temperature for 10 minutes and pelleted by centrifugation at maximum speed for 20 minutes. The supernatant was aspirated and the DNA pellet was washed carefully with 1 ml ice-cold 70% ethanol. If the pellet was disrupted the sample was centrifuged at maximum speed for 10 minutes. The ethanol was removed and the DNA pellet was dried under laminar flow for 5 to 10 minutes. The purified DNA was suspended in 30 to 50 μl TE buffer. The quality of the DNA was determined by 0.8 % agarose gel electrophoresis overnight at 20 V. HMW DNA was indicated above the 23.13 kb mark of λHindIII DNA marker.

## EXAMPLE 2

*Metagenomic Library Construction*

The randomly sheared DNA ends of the HMW DNA was end-repaired using the End It™ DNA repair enzyme components from the pCC2FOS CopyControl™ fosmid library production kit (Epicentre). In an 80 μl reaction volume 15 μg of agarose gel purified HMW DNA and the kit components according to the manufacturer's recommendations. The reaction was incubated at room temperature for 45 minutes then heat inactivated at 70°C for 10 minutes. Residual enzyme was removed by phenol/chloroform extraction. The volume was increased to 500 μl by the addition of TE buffer. An equal volume of phenol: chloroform: isoamyl alcohol (25:24:1 v/v) (PCI) was added to the TE buffer. The phases were separated by centrifugation. The aqueous phase was removed and the DNA was precipitated by adding a 1/10 sample volume of 3 M sodium acetate (pH 7.0) and 2.5 volumes of absolute ethanol to the supernatant precipitated at room temperature for 10 minutes. The precipitated DNA was harvested by centrifugation at maximum speed for 20 minutes. The supernatant was aspirated and the DNA pellet was carefully washed with 1 ml ice-cold 70% ethanol. If the pellet was disrupted the sample was centrifuged at maximum speed for 10 minutes. The ethanol was removed and the DNA pellet was dried under laminar flow for 5 to 10 minutes. The purified DNA was suspended in 20 μl TE buffer. The DNA concentration was measure fluorometrically by the Qubit™ fluorimeter (ThermoFisher Scientific).

*Ligation, phage packaging and average insert size determination*

The recommended amount of HMW DNA (200 ng) was used in the pCC2FOS vector ligation protocol using the Fast Link™ ligase (Epicentre) from the CopyControl™ kit. The reaction was incubated at room temperature for 4 hours followed by heat inactivation at 70°C for 10 minutes. The phage packaging reaction was conducted using Copy-Control™ kit according to the manufacturer's instructions. The library titre was determined by serial dilution and was calculated according to Equation 2-1. The average insert size of the library was determined by restriction enzyme digestion of 50 clones by digesting 10 μg fosmid with *EcoRI* and *HindIII* restriction enzymes. The fosmids were extracted according to Example 4. The restriction digest was electrophoresed on 0.8% TAE agarose for 16 hours at 30 V.

*Fosmid linearization and dephosphorylation*

The pCCERI vector was linearized by digestion with 100 U *BstZ17I* (Fermentas) of 20 μg pCCERI for 3 hours at 37°C in a minimum volume of 100 μl. The restriction enzyme digest was divided into 1 μg aliquots and dephosphorylated by rAPID Alkaline Phosphatase (Roche) for 10 minutes at 37°C. The reactions were pooled and an equal volume of CI (24:1) was added and centrifuged at 16 000 *x g* for 15 minutes. The aqueous phase was removed and 1/10 volume of 3 M sodium acetate (pH 7) and 2 x volume absolute ethanol was added. The DNA was precipitated on ice for 10 minutes then centrifuged at 16 000 x g for 20 minutes. The pellet was washed with 1 ml 70% ethanol. The DNA was centrifuged at 16 000 *x g* for 5 minutes. The sample was dried and suspended in 30 μl TE.

*Ligation of HMW DNA into pCCERI and phage packaging*

End-repaired HMW DNA was ligated into the pCCERI fosmid using the Fast Link Ligase (Epicentre), but T4 ligase from other suppliers can be substituted. The ligation was performed using 500 ng of dephosphorylated pCCERI vector and 250 ng end-repaired HMW DNA, incubated for 4 hours at room temperature followed by heat inactivation at

70°C for 10 minutes. The 10 μl ligated reaction was used for the phage packaging reaction into *E. coli* EPI300 containing the pER1.3.50.2 helper plasmid. Transfected colonies were selected on Lysogeny Broth (LB) agar containing 12.5 μg/ml chloramphenicol and 50 mg/ml kanamycin as selective antibiotics. The library titre was determined according to Equation 2-1 and stored as unamplified library in 20% glycerol.

*Conjugation of pCCERI metagenomic libraries into* Pseudomonas putida *MBD1*

*P. putida* MBD1 was inoculated into 5 ml LB media containing 50 μg/ml kanamycin and incubated overnight at 30°C shaking at 125 rpm. The overnight culture was diluted 1:25 in LB containing 10 mM MgCl2 and incubated for 2 hours at 30°C shaking at 125 rpm then heat shocked at 42°C for 10 minutes. After which, 2 ml of the culture was added to 40 ml LB containing 10 mM MgCl2 and 0.02% L-arabinose. A 100 μl of the culture was dispensed into 96- microwell plates. An overnight culture of *E. coli* containing the pCCERI metagenomic library was grown in LB media containing 12.5 μg/ml chloramphenicol, 50 μg/ml kanamycin and 0.02% L-arabinose incubated at 37°C shaking at 225 rpm. A 10 μl volume of overnight *E. coli* culture was dispensed into the 96-well plates containing the 100 μl *P. putida* MBD1 by 96-pin replicator stamp. The 96-well plates were incubated without shaking overnight at 30°C. The conjugants were transferred by replicator stamp onto M9-benzoate agar containing 30 μg/ml apramycin (Sigma). The plates were incubated overnight at 30°C. The colonies were transferred to the various testing agar or culture media for biosurfactant activity screening.

## EXAMPLE 3

*Biosurfactant Detection*

The paraffin droplet spray method of Burch *et al.* (2010) was used to detect the presence of biosurfactants around bacterial colonies arrayed on agar. The *E. coli* metagenomic libraries were arrayed onto LB agar (12.5 μg/ml chloramphenicol, 50 μg/ml kanamycin, 0.02% L-arabinose) and incubated overnight at 37°C. The P. *putida* MBD1 metagenomic conjugants were arrayed using a 96-pin replicator stamp onto *Pseudomonas* Selective Agar (Merck) containing 30 μg/ml apramycin and incubated overnight at 30°C. The *S. lividans* Δact Δred were arrayed on R5 agar and incubated overnight at 30°C. An airbrush (Airbrush compressor kit, AirCraft Pneumatic Systems) was used to apply a fine mist of paraffin onto the agar plate between 15 and 20 Bar. Biosurfactant halos were immediately visible under an indirect light source.

Alternatively a microplate assay was used to detect the presence of a biosurfactant, in this method metagenomic clones were cultured in M9 media containing 2% glucose and incubated at 37°C for three days for *E. coli* libraries and 30°C for three days for *P. putida* MBD1 libraries in deep-well 96-well plates. A 100 μl of culture supernatant was dispensed to 96-well flat bottomed plates. The plate was viewed using grid paper where the presence of biosurfactants is indicated by the reducing in grid square sizes on the grid paper.

In yet an alternative method M9 agar was supplemented with 0.02% cetyltrimethylammonium (CTAB) agar and methylene blue was used to detected extracellular anionic biosurfactants produced by *P. putida* MBD1 (Siegmund and Wagner 1991). Minimum media agar was supplemented with glucose, CTAB and methylene blue. Bacterial colonies were arrayed on the CTAB-agar plates and incubated at 30°C for *P. putida* MBD1 and at 37°C for *E. coli* EPI300 until a blue halo was observed.

Several standard biosurfactant screening methods were tested concurrently to evaluate their suitability as high throughput methods for screening metagenomic libraries. These included the microplate assay, the drop collapse assay, paraffin droplet spray and on CTAB methylene blue agar (Burch *et al.* 2010; Walter *et al.* 2010). No positive results were obtained using the drop collapse or microplate assays. Considering the time, effort and success, the paraffin droplet spray method was the most convenient and high throughput method for screening large metagenomic libraries. With no prior preparation, the colonies could be arrayed on agar and be screened directly, whereas liquid assays required the recovery of the culture supernatant for the drop collapse or microplate methods. Furthermore, Burch *et al* (2011) determined the droplet spray method was more sensitive than liquid assays, because it was able to identify biosurfactants that are not readily soluble in the culture media, therefore would be missed by liquid assays such as the microplate and drop collapse method (Burch *et al.* 2011).

Background biosurfactant activity produced by the host will result in false positives during the library screening process. Both *E. coli* and *P. putida* did not produce background biosurfactant activity when tested. As a result the inventors used *P. putida* as the screening host.

*Metagenomic library characteristics*

A library constructed in *E. coli* Epi300 using lake sediment mDNA and using the pCC2FOS commercial cloning

vector, resulted in a metagenomic library consisting of 261 000 clones at an average insert size of 31.1 kb (Figure 1). These libraries were cloned into a shuttle fosmid, pCCERI (O'Gara 2012) comprising of 84 000 clones.

The pCCERI fosmid uses the φC31 integration system of a *Streptomyces* phage to integrate itself into the genomes of the engineered *Pseudomonas putida* strain MBD1, derived from *P. putida* KT2440 (Martinez *et al.* 2004), and allows the transfer of large-insert DNA libraries from *E. coli* into these alternative bacterial hosts. Expansion of the bacterial host range broadens promoter recognition and allows expression of genes from metagenomic inserts that are silent in the pCCFOS/*E. coli* expression system. This enabled the mDNA to be screened using the parallel host functional metagenomics strategy that enables more of the mDNA to be interrogated by a diversity of metabolic environments (Craig *et al.* 2010).

*Screening of mDNA from Lake Sediment Soils (LSSpCCERI) for biosurfactant activity*

After screening 40 000 *E. coli* LSSpCCERI clones, no biosurfactant activity was detected by any method.

Concurrently, the fosmids were transferred into *P. putida* MBD1 by conjugation, achieving 97% conjugation efficiency.

Employing the paraffin spray method, a putative biosurfactant producing clone in *P. putida* (LSSpCCERI) was identified arrayed on *Pseudomonas* Selective agar. The clone (named *P. putida* MBD1-E54F3) demonstrates the characteristic surfactant halo, due to the biosurfactant surrounding the clone interacting with the paraffin (Figure 2). No biosurfactant activity was detected using the microwell plate, drop collapse or CTAB/methylene blue assay for this clone, thus it is likely that the biosurfactant produced is hydrophobic (Burch *et al.* 2011). It is also not anionic due to lack of activity on the CTAB/methylene blue agar assay, where anionic biosurfactants form an insoluble complex with CTAB and is visualised by the methylene blue dye aggregation.

The absence of activity in *E. coli* EPI300 and manifestation of activity in *P. putida* vindicates the parallel host screening strategy for functional metagenomic screening over the predominant approach, where libraries prepared in the pCCFOS vector are only screened in *E. coli.*

*Emulsification capacity after 24 hours ($EC_{24}$)*

The $EC_{24}$ method was adapted from Cooper and Goldenberg (Cooper and Goldenberg 1987). An equal volume of paraffin and culture supernatant was vortexed at high speed for 2 minutes. The height of the stable emulsion layer was measured after 24 hours. The emulsion index ($EC_{24}$) was calculated using the following equation:

$$EC_{24} = H_{emulsion}/H_{total} \times 100\%$$

The biosurfactant activity of *P. putida*-E54F3 was confirmed by determining the $EC_{24}$ of the culture supernatant. A water-in-oil emulsion with an $EC_{24}$ of 70% was formed (Figure 3), further confirming a potentially hydrophobic biosurfactant with a low HLB value. The emulsion remained stable at room temperature for several months. The *P. putida* control formed a slight emulsion but was not as stable or as significant as the *P. putida*-E54F3, while no emulsion was formed by *E. coli*-E54F3 (Figure 4).

## EXAMPLE 4

*Plasmid DNA extraction methods*

Fosmid DNA from the metagenomic libraries were extracted using either the Midiprep Plasmid Extraction Kit (Qiagen) or the SDS lysis method (Sambrook *et al.* 2001). The same methods were used for general plasmid extractions in LB media supplemented with the appropriate antibiotic.

*Fosmid extraction using the Qiagen Midiprep Plasmid Extraction Kit*

A freshly grown colony of *E. coli* containing the fosmid was inoculated into 50 ml LB with 12.5 μg/ml chloramphenicol and 0.02% L-arabinose in an Erlenmeyer flask. The DNA was extracted using the Midiprep Plasmid Extraction kit (Qiagen) according to the manufacturer's instructions.

*Fosmid extraction using the SDS lysis method*

A freshly grown colony of *E. coli* or *P. putida* was inoculated into 10 ml LB with 12.5 μg/ml chloramphenicol

and 0.02% L-arabinose in an Erlenmeyer flask. The culture was incubated overnight at 37°C shaking at 250 rpm to ensure good aeration. The culture was transferred to 15 ml Falcon tube and centrifuged at 6000 *x g* for 10 minutes at 4°C. The supernatant was removed and Falcon tube was inverted on a paper towel and dried. The cell pellet placed on ice and 1 ml cooled Glucose/Tris/EDTA (GTE) solution (50 mM glucose, 25 mM Tris-HCl, pH 8.0, 10 mM EDTA) was added. The pellet was suspended in the GTE solution completely before continuing. One millilitre SDS/NaOH solution (0.2 N NaOH, 1% SDS) was added and inverted gently twice. Finally, 1 ml 5 M sodium acetate (pH 5.5) was added to the solution, was inverted twice and incubated on ice for 15 minutes, inverting every 5 minutes. The solution was transferred to 2 x 2 ml microcentrifuge tubes and centrifuged at 16 000 *x g* for 30 minutes at 4°C. The supernatant was divided into 3 x 2 ml microcentrifuge tubes containing 1 volume of isopropanol. The DNA was precipitated by incubation at room temperature for 15 minutes. The DNA was collected by centrifugation at 16 000 *x g* at 10-15°C for 30 minutes. The supernatant was aspirated and 1 ml ice-cold 70% ethanol was added without disturbing the pellet. The ethanol was poured off and the DNA pellet dried under laminar flow. The DNA was suspended in 500 μl TE buffer and added to 500 μl Cl (24: 1). The sample was incubated on ice for 10 minutes after which it was centrifuged at 16 000 *x g* for 10 minutes. The aqueous phase was transferred to 0.6 volumes of isopropanol. The DNA was precipitated for 30 minutes at room temperature. DNA was pelleted by centrifugation at 16 000 *x g* for 10 minutes. The supernatant was aspirated and 1 ml 70% ethanol was carefully added to wash the pellet. The 70% ethanol was removed and the pellet was dried under laminar flow. The pellet was dissolved in 50 μl TE buffer.

## EXAMPLE 5

*Preparation of competent cells*

Electrocompetent *E. coli* bacterial cells were prepared by a series of washing steps with 10% glycerol. A fresh colony of *E. coli* on LB agar was used to inoculate 10 ml LB media, which was incubated overnight at 37°C shaking at 125 rpm. A 5 ml aliquot of the overnight culture was used to inoculate 500 ml LB media and incubated at 37°C shaking at 225 rpm until the culture reached an optical density (OD) at 600 nm of between 0.5 and 0.7. The cells were pelleted by centrifugation at 4000 *x g* for 15 minutes at 4°C. The cells were suspended in 250 ml ice-cold 10% glycerol, followed by centrifugation at 4000 *x g* at 4°C for 15 minutes. The supernatant was removed and the cells suspended in 125 ml ice-cold 10% glycerol then centrifuged at 4000 *x g* at 4°C for 15 minutes. The cell pellet was suspended in 20 ml 10 % glycerol and transferred to a 50 ml falcon tube then pelleted by centrifugation at 4000 *x g* at 4°C for 15 minutes. The cells were finally suspended in 2 ml ice-cold 10% glycerol to obtain a culture of between 1 to 3 x $10^{10}$ cells/ml. The cell suspension was divided into 200 μl aliquots and stored at -80°C.

## EXAMPLE 6

*DNA sequencing*

Fosmid ends and Polymerase Chain Reaction (PCR) amplicons were sequenced at the University of Stellenbosch Sequencing Facility using an ABI PRISM 377 Automated DNA Sequencer in both the forward and reverse directions. Sequences are evaluated using CLC Main Workbench 7 software (CLCbio).

Sanger sequencing of the fosmid ends was performed to analyse the metagenomic libraries for any cloning bias. Randomly selected fosmids were sequenced and open reading frames (ORFs) situated at the fosmid ends were searched by BLASTN against the NR database. Each end-sequencing result displayed different end-sequences for each fosmid, as well as different bacterial species origin for the ORFs identified. Based on these results there appeared to be no obvious cloning bias in the LSSpCCFOS2 libraries.

*Illumina MiSeq fosmid insert sequencing*

The fosmid demonstrating activity was extracted as set out above and submitted to the University of the Western Cape Sequencing Facility. The sequencing library was prepared with 1 ng fosmid DNA using the Nextra XT DNA Library Preparation Kit (Illumina) and the sequencing reaction was performed on the Illumina MiSeq platform.

*Fosmid sequence assembly*

The paired-end sequence reads were assembled into contiguous sequences using the CLC Genomics Workbench version 6.5 software (CLCbio). Prior to *de novo* sequencing, the reads were trimmed to remove low quality reads and adapters using the following parameters; quality score = 0.05, maximum ambiguous bases = 3, minimum sequence length 50 nucleotides and removal of adapter sequences. Contigs were constructed by *de novo* assembly using the

following parameters; mismatch cost = 2, insertion cost = 3, deletion cost = 3, length fraction = 0.95, similarity fraction 0.95 and minimum contig length of 1000 bp. The largest contig assembled was at the predicted size and was used for further bioinformatics analysis.

*Annotation of the fosmid sequence*

The FASTA formatted contig designated E54F3 was assigned open reading frames using the FGENESB (Solovyev and Salamov 2011a) online pipeline (www.softberry.com) using the following parameters; Table of Genetic Code = 11, closest organism = ARCHAE BACTERIAL generic.

The ORFs assigned and translated by FGENESB were annotated by similarity searches with by the BLASTP (Altschul *et al.* 1997) using the Non-Redundant (NR) protein database (Pruitt *et al.* 2002) at NCBI using the default parameters. The top hit returned ORF was used to assign the molecular function to that ORF. The putative biosurfactant producing ORF was used for hidden Markov Model (HMM) based alignment using Reference Proteome database using the HMM web server (Finn *et al.* 2011; Finn *et al.* 2015).

Evaluation of the functional annotations for E54F3 revealed no obvious candidate ORF that might contribute to biosurfactant activity. Either the ORF(s) responsible for the observed biosurfactant activity is one of the hypothetical proteins or the sequences from the archival databases were inaccurately annotated. Furthermore the activity could be due to complementation of an endogenous biosurfactant pathway by an ORF on E54F3. In order to establish which scenario was the case, Tn5 transposon mutagenesis was conducted in vitro on the E54F3 fosmid and biosurfactant negative clones selected.

Of the three clones mapped for transposon insertions, all mapped within ORF32, annotated as a putative haemolysin belonging to the COG3176 category (putative haemolysins by function prediction only), a poorly characterised COG (Table 1). However, when tested for haemolytic activity by blood agar assay, both *P. putida* MBD1 and the *P. putida* E54F3 was not haemolytic, thus the annotation was inconsistent with the activity.

Table 1: The BLASTP hit table for E54F3 ORF32 with the highest similarity belonging to a putative haemolysin. ORF32's comparison to biochemically confirmed OlsBs are shown in the last two rows.

| Description | Total score | Query cover | Expect Value | Identity | Accession |
|---|---|---|---|---|---|
| haemolysin [Methylobacterium sp. Leaf113] | 292 | 93% | 1,00E-93 | 56% | WP_ 056179419.1 |
| haemolysin [Methylobacterium sp. Leaf104] | 291 | 93% | 3,00E-93 | 56% | WP_ 056466473.1 |
| haemolysin [Methylobacterium sp. Leaf117] | 289 | 93% | 2,00E-92 | 55% | WP_ 056484082.1 |
| Putative haemolysin [Candidatus Phaeomarinobacter ectocarpil | 288 | 93% | 2,00E-91 | 55% | CDO59826.1 |
| hypothetical protein [Parvibaculum lavamentivoransl | 286 | 90% | 4,00E-91 | 57% | WP_ 012112455.1 |
| conserved hypothetical protein [Methylobacterium extorquens AM1] | 284 | 93% | 2,00E-90 | 56% | ACS42468.1 |
| haemolysin [Methylobacterium extorquens] | 283 | 93% | 2,00E-90 | 56% | WP_ 041358828.1 |
| Ornithine acyl-ACP N-acyltransferase *[Sinorhizobium meliloti* 10211 | 251 | 92% | 9,00E-82 | 50% | WP_ 010968546.1 |
| Conserved hypothetical *aeruginosa* PA011 | 82 | 84% | 7,00E-19 | 32% | WP_ 003093970.1 |

Previous studies also found an inconsistency with the COG3176 functional assignment as a putative haemolysin (Gao *et al.* 2004). A putative haemolysin (SMc01127) from *Sinorhizobium meliloti* is one of the representative sequences of the COG3176 group (Table 2), but was biochemically confirmed as an ornithine acyl-ACP N-acyltransferase (OlsB) by radiolabelled ornithine incorporation into the ornithine lipid (OL) product as well as by ornithine lipid mutant complementation experiments of the ornithine acyltransferase gene (Gao *et al.* 2004). The initial phylogenetic analysis of

COG3176 indicates either it contains protein sequences with two distinct functions, haemolysin activity and ornithine acyl-ACP N-acyltransferase activity or these proteins are bifunctional.

Table 2: The representative sequences of the COG3176 category obtained from the CDD database on NCBI.

| **NCBI Reference** | **Name** | **Bacteria** | **AA sequence size** |
|---|---|---|---|
| NP_233035.2 | Hemolysin | *Vibrio cholerae* 01 biovar El Tor str. N16961 | 605 |
| NP_353376.2 | Conserved hypothetical protein | *Agrobacterium fabrum* str. C58 | 300 |
| WP_ 011005405.1 | MULTISPECIES: hypothetical protein | *Brucella* | 281 |
| NP_540717.1 | Hypothetical protein BMEI1800 | *Brucella melitensis* bv. 1 str. 16M | 281 |
| NP_ 422327.1 | Acyltransferase | *Caulobacter crescentus* CB15 | 235 |
| NP_337627.1 | Hypothetical protein MT3111 | *Mycobacterium tuberculosis* CDC1551 | 281 |
| NP_253040.1 | Hypothetical protein PA4350 | *Pseudomonas aeruginosa* PAO1 | 251 |
| NP_519659.1 | hypothetical protein RSc1538 | *Ralstonia solanacearum* GMI1000 | 278 |
| NP_217543.2 | GCN5-like N-acetyltransferase | *Mycobacterium tuberculosis* H37Rv | 246 |
| NP_384499.1 | Hypothetical protein SMc01127 | *Sinorhizobium meliloti* 1021 | 296 |
| NP_ 484272.1 | Hypothetical protein alr0228 | *Nostoc* sp. PCC 7120 | 267 |
| NP_104372.1 | Hypothetical protein mlr3216 | *Mesorhizobium loti* MAFF303099 | 312 |

*Multiple sequence alignment and phylogenetic analysis*

The biosurfactant candidate ORF32, annotated hemolysin/ornithine acyl-ACP N-acyltransferase, was aligned to *olsB* annotated protein sequences found on NCBI using MAFFT version 7 (Katoh and Standley 2013) using the default parameters; scoring matrix for amino acid sequences = BLOSUM62, gap opening penalty = 1.53. The MAFFT alignment was visualised using UGENE version 1.20.0 software (Okonechnikov *et al.* 2012).

The MAFFT output was converted to PHYLIP 4 format and upload to ATGC-PHYML (http://www.atgc-montpellier.fr/phyml/) using the PhyML version 3.0 software (Guindon *et al.* 2010) using the LG substitution model and aLRT SH-like fast likelihood-based method. The tree was constructed using FigTree version 1.4.2 (http://tree.bio.ed.ac.uk/software/figtree/).

All sequences in the OlsB-like clade contained conserved domains belonging to the lysophospholipid acyltransferase (LPLAT) protein superfamily, whose members incorporate acyl groups from either acyl-CoA or acyl-acyl carrier proteins into acceptors such as glycerol 3-phosphate, dihydroxyacetone or lyso-phosphatidic acid (Shindou *et al.* 2009). LPLAT members are involved in glycerophospholipid biosynthesis in both *de novo* and remodelling pathways. A key feature of this superfamily is the invariant histidine and aspartic acid residues found in a conserved motif HX4D configuration. The H-residue is essential for glycerolipid acyltransferase activity while D substitution can lead to either elimination of activity or significant reduction. Without this motif the protein might not have any acyltransferase activity (Heath and Rock 1998). A multiple sequence alignment showed that the Hx4D conserved motif was present in all sequences of the OlsB-clade, but absent in the proteins belonging to the haemolysin-clade (Figure 5), suggesting that haemolysin A representatives likely do not possess acyltransferase activity.

E54F3 ORF32 cosegregated with the putative haemolysin from *Methylobacterium* Leaf113 and *Parvibaculum lavamentivorans*, also contained the HX4D motif (H149 and D154; in diagram H386 and D 391 using *V. cholerae* amino acid numbering) (Figure 5). The sequences that represent the COG3176 category also contained the HX4D motif except the hypothetical protein AL0228 of Nostoc sp. PCC 7120 and the haemolysin of *Vibrio cholerae* 01 biovar El Tor str. N16961. The *Nostoc* sp. protein sequence contained the invariable H residue but not the D residue while the *Vibrio choleae* protein sequence did not contain the HX4D motif at all. The length of the *V. cholerae* haemolysin protein sequence is also significantly longer (605 amino acids) while the rest of the sequences average 302 amino acids in size. These initial analyses provide motivation to re-evaluate the COG3176 category to accurately provide a functional category.

Disruption of ORF32 eliminates the biosurfactant activity observed *in vivo*, but the ambiguous bioinformatic annotation casts doubt on its functional classification. ORF32 is similar to sequences belonging to the COG3176 group annotated as putative haemolysins and has the highest similarity to a conserved hypothetical protein from *P. lavamentivorans* and other putative haemolysins. However, previous biochemical experiments show that some protein sequences belonging to the COG3176 group have ornithine acyl-ACP N-acyltransferase (OlsB) activity rather than haemolysin activity. OlsB activity would be the expected activity if ORF32 is responsible for the synthesis of a biosurfactant molecule. Furthermore, the presence of the Hx4D motif further suggests that ORF32 is an acyltransferase.

## EXAMPLE 7

OlsB catalyses the first step in the two-step pathway of ornithine lipid (OL) synthesis, where the non-proteinogenic amino acid, ornithine, is N-acylated by the transfer of the β-hydroxyfatty acid from its Acyl Carrier Protein (ACP) to ornithine by an amide linkage to form the single hydrocarbon chained, LOL (Gao *et al.* 2004). A second β-hydroxyfatty acid is coupled to the β-hydroxyl group of the first fatty acid in an ester linkage by the lyso-ornithine lipid dependent O-acyltransferase, OlsA (Weissenmayer *et al.* 2002) to form the double fatty acid chained OL (Figure 6). Ornithine lipids are water-in-oil emulsifiers due to their significant hydrophobic portion, LOL biosurfactant properties have not been previously investigated.

Ornithine lipid production is widely distributed amongst eubacteria, (López-Lara *et al.* 2003; Geiger *et al.* 2010), and about 25% of sequenced bacterial genomes are predicted to be OL producers (Vences-Guzmán *et al.* 2014). This percentage will have to be revised since the majority of the predicted sequences may have been misannotated as putative hemolysins on NR databases. In many bacteria the *olsB* and *olsA* occur in an operon, though it is not an absolute requirement (Gao *et al.* 2004). In members of the α-proteobacteria and β-proteobacteria the *olsB*-like and *olsA*-like sequences can be separated by large chromosomal distances (Gao *et al.* 2004). The apparent lack of an *olsA*-like sequence on the E54F3 fosmid as well as the E54F3 ORF32 similarity to α-proteobacteria *olsB* sequences could suggest that if ORF32 is responsible for LOL production, the *olsA* is not linked to the *olsB* on the genome of the originating host. No *olsA*-like gene is visible immediately adjacent to the closely-related putative *olsB* genes identified in the genomes of *Methylobacterium* sp. Leaf113 and *P. lavamentivorans* DS1. Therefore there are two hypothetical ornithine containing lipid structures that could be produced by *P. putida* MBD1-E54F3, a single fatty acid chain LOL produced by ORF32's hypothesised OlsB activity or the double fatty acid chained, OL, formed by ORF32 complementation of an endogenous *P. putida* OlsA-like enzyme.

To assess the expression and the characterisation of the biosurfactant produced by *P. putida* MBD1-E54F3, the following analyses were conducted: cDNA synthesis, expression of ORF32 under the control of the T7-promoter in *E. coli*, the isolation of ornithine containing lipids from *P. putida* MBD1+E54F3; TLC analysis of the extracted biosurfactant; and mass spectrometry analysis of biosurfactant extracts of *P. putida* MBD1-E54F3.

*Endogenous* olsA *and* olsB*-like sequences in* P. putida *MBD1*

During the course of the bioinformatic investigation of *olsB* sequences found in the literature, *Pseudomonas aeruginosa* PA01 was one of the two biochemically confirmed ornithine lipid producers containing *olsB* and *olsA*-like gene sequences within its genome (Lewenza *et al.* 2011). The *P. aeruginosa* PA01 genes were PA4350, a hypothetical protein (putative *olsB*) and PA4351 an acyltransferase (putative *olsA*). Since *P. putida* is a close relative of *P. aeruginosa,* the presence of *olsA* and *olsB*-like gene sequences was suspected even though under the conditions tested, no residual biosurfactant activity was detected.

A BLASTP analysis of the *P. putida* KT2440 genome (the parent strain of *P. putida* MBD1) (Martinez *et al.* 2004) using the PA4350 and PA4351 protein sequences revealed two sequences with relatively high similarity. PA4350 the putative *OlsB* had 79% protein sequence identity to PP0924, which is annotated as a hypothetical protein in the *P. putida* KT2440 genome. While PA4351 had 85% protein sequence identity to PP0923, which is annotated as an acyltransferase in the *P. putida* KT2440 genome. The presence of both *olsB* and *olsA*-like genes in *P. putida* is curious because no background biosurfactant production was observed during the metagenomic screening of thousands of *P. putida* trans-conjugates using the paraffin spray method. ORF32 is hypothesised to produce LOL, which could be solely responsible for the biosurfactant activity observed in *P. putida* MBD1-E54F3. However, the presence of an *olsA*-like gene could result in ORF32 complementing the entire pathway to produce the double fatty acid chained OL. The combination of ORF32 and the putative *olsA* of *P. putida* could produce OL over and above what the *P. putida* normally produces, thereby surpassing the limit of detection of the paraffin spray method.

*Overexpression* of *ORF32 in* E. coli *BL21*

ORF32 was amplified using ORF32_NdeI and ORF32_NotI primers (Table 3) and cloned into pET21a(+) (Novagen). The pET21a-ORF32 was transformed into electrocompetent *E. coli* BL21 (DE3) codon plus by electroporation and selected on LB agar containing 100 μg/ml ampicillin.

A freshly streaked colony of *E. coli* BL21-ORF32 was used to inoculate 5 ml LB media containing 100 μg/ml ampicillin and incubated at 37°C overnight shaking at 225 rpm. One tenth of the overnight culture was used to inoculate LB media containing 100 μg/ml ampicillin and increasing concentrations of L-ornithine (Sigma). The culture was incubated at 30°C shaking at 225 rpm until it reached an $OD_{600nm}$ ~ 0.5. ORF32 was induced by the addition of 1 mM isopropyl β-D-1-thiogalactopyranoside (IPTG) and incubated at 30°C overnight shaking at 225 rpm.

Table 3: Primers used in this study

| Primer | Description | Sequence | SEQ ID | Source |
|---|---|---|---|---|
| E9F | Universal bacterial 16S rRNA | GAGTTTGATCCTGGCTCAG | SEQ ID NO: 3 | Hansen et al., 1998 |
| U151OR | Universal bacterial 16S rRNA | GGTTACCTTGTTACGACTT | SEQ ID NO: 4 | Reysenbach and Pace, 1995 |
| OlsB873F | Amplifies the E54F3 ORF32 | GTGGGCGAACAGAGGAAAAAG | SEQ ID NO: 5 | This work |
| OlsB873R | Amplifies the E54F3 ORF32 | TTTTTGTCAGCGGCTTTGGC | SEQ ID NO: 6 | This work |
| OlsB1029F | Amplifies E54F3 *olsB* ORF plus upstream region | CGAATGCCATCCCCACATCA | SEQ ID NO: 7 | This work |
| OlsB1029R | Amplifies E54F3 *olsB* ORF plus upstream region | GCTTTTTGTCGGCGGTCTTT | SEQ ID NO: 8 | This work |
| ORF32_NdeI | Amplifies E54F3 ORF32 with NdeI restriction site | GGAATTCcatatgTTGGTAAATATTT ACGAGCC | SEQ ID NO: 9 | Designed by Lungelo Mandyloli |
| ORF32_NotI | Amplifies E54F3 ORF32 with NotI restriction site | ATAAGAATgcggccgcGCGGCTTTG GCCGAAACGGC | SEQ ID NO: 10 | Designed by Lungelo Mandyloli |

In order to determine if *E. coli* is capable of producing ORF32 dependent biosurfactant activity, the ORF32 sequence was sub-cloned into pET21a and expressed in *E. coli* BL21 under the control of the T7 promoter rather than its own promoter. Since ORF32 was linked by *in silico* analysis to ornithine acyl-ACP N-acyltransferases, the expression of ORF32 was conducted with increasing concentrations of ornithine.

ORF32 expression under the control of the T7 promoter resulted in the production of biosurfactant activity in an ornithine dose dependent manner in *E. coli* BL21. Increased foam production (Figure 7A) and emulsification (Figure 7B) was observed with increasing ornithine concentrations. *E. coli* is capable of endogenous ornithine synthesis (Lee and Cho 2006) therefore the production of ornithine containing lipids is possible if the required genes are available. The amount of biosurfactant made might be limited by the level of ornithine available to *E. coli.* When repeating the experiment in a smaller volume (100 ml *E. coli* pET21a-ORF32 LB media cultures, including the cultures with no IPTG induction and no ornithine supplementation), surface tension reduction properties were displayed, whereas the control *E. coli* BL21 cultures had no activity (Figure 8).

The emulsification stability after 24 hours was tested and all the *E. coli* pET21a-ORF32 cultures produced stable emulsions irrespective of the presence or absence of 1 mM Isopropyl β-D-1-thiogalactopyranoside (IPTG) or 10 mM ornithine (Figure 9). The *E. coli* BL21 culture controls emulsified paraffin poorly. An $EC_{24}$ > 50% was observed in *E. coli* pET21a-ORF32 culture supernatants for every condition of IPTG and ornithine supplementation (Table 4). The pET21a-ORF32 cultures supplemented with 10 mM ornithine showed > 60% $EC_{24}$, which is significantly higher than the

0 mM ornithine cultures. pET21a-ORF32 cultures supplemented with 10 mM ornithine and 1 mM IPTG showed > 66.7% $EC_{24}$ An $EC_{24}$ < 20% was observed for *E. coli* BL21 culture supernatants (Table 4). Furthermore, the cultures containing 10 mM ornithine were more resistant to physical disruption of their emulsion by vortex than the cultures without ornithine (Figure 10). The results were indicative of increased concentrations of biosurfactant, the production of which was linked to ornithine supplementation. The ability to produce biosurfactant activity in the absence of exogenous ornithine indicates the endogenously produced ornithine was sufficient to produce biosurfactant activity in *E. coli,* but supplementation of ornithine increased the response in a dose-dependent manner.

Table 4: The emulsification stability index after 24 hours for the culture supernatants of *E. coli* BL21 without pET21a-ORF32 and with pET21a-ORF32.

| **Name** | **1 mM IPTG** | **10 mM Ornithine** | **$EC_{24}$** |
|---|---|---|---|
| *E. coli* BL21 | - | - | 18.75 % |
| *E. coli* BL21 | + | - | 6.25 % |
| *E. coli* BL21 | + | + | 18.75 % |
| *E. coli* pET12a-ORF32 | - | - | 56.25 % |
| *E. coli* pET21a-ORF32 | + | - | 50 % |
| *E. coli* pET21a-ORF32 | - | + | 60% |
| *E. coli* pET21a-ORF32 | + | + | 62.5%-66.7% |

Microscopic evaluation of the emulsions further revealed an ornithine concentration dependent pattern. Equal volumes of polydispersed emulsions (Figure 9) were visualised microscopically at 10 x magnification (Figure 11). The *E. coli* BL21 (Figure 11, A) emulsions were visually different from what was obtained with the *E. coli* BL21-ORF32 cultures (Figure 11, B and C). The *E. coli* BL21 emulsions formed an arrangement of smaller droplets within a large droplet. *E. coli* BL21-ORF32 cultures without ornithine or IPTG supplementation formed larger uniform droplets, whereas uniform but smaller droplets were visualised with the *E. coli* BL21-ORF32 cultures with 10 mM ornithine and 1 mM IPTG supplementation. Smaller droplet size is an indicator of increased emulsifier concentration (McClements 2015) therefore *E. coli* BL21-ORF32 supplemented with ornithine and IPTG potentially has a higher concentration of the biosurfactant than in the *E. coli* BL21-ORF32 cultures without ornithine and IPTG supplementation.

RNA analysis indicated that ORF32 was not expressed in *E. coli* EPI300-E54F3 when under the control of its own promoter. When ORF32 is placed under the control of the T7 promoter, both surface tension reduction (Figure 8) and increased emulsion stability (Figure 9, Table 4) was observed. We can therefore conclude *E. coli* is capable of accumulating an ornithine concentration dependent biosurfactant if ORF32 is expressed under the control of the T7 promoter. This also provided support for the *in silico* analysis suggesting that the product of ORF32 expression is an ornithine containing biosurfactant.

*E. coli* has no apparent OlsA (Lopez-Lara *et al.* 2003), it is therefore presumed that expression of ORF32 yields LOL. Furthermore, based on the similarly structured N-acyl amino acids (Brady and Clardy 2000; Peypoux *et al.* 2004) LOL has probable emulsification and surface tension reduction properties. It might be possible that *E. coli* has an O-acyltransferase homologue that can catalyse the ester bond formation to produce OL from the ORF32's LOL. However, without further experimental investigation, it is not possible to establish if OL is actually produced.

## EXAMPLE 8

*Total lipid extraction in low and high phosphate culture conditions*

Ornithine lipid was detected by TLC of the total lipid extraction tested under how and low conditions.

P. putida *E54F3 and MBD1 cultured in high and low phosphate media*

The *P. putida* was cultured in 50 ml Basal Media 2 supplemented with 1600 μM phosphate buffer (pH 7.2) for high phosphate media or with 400 μM phosphate buffer for low phosphate media (Mulcahy *et al.* 2010). The media containing the appropriate antibiotics was inoculated with a freshly cultured single colony of *P. putida* MBD1 or E54F3 and incubated overnight 30°C shaking at 250 rpm. *E. coli* EPI300 cannot be cultured using this media. The overnight culture was either used total lipid extraction.

Previous studies of OL production in *P. aeruginosa* PA01 (Lewenza *et al.* 2011) and *Sinorhizobium meliloti*

(Geiger *et al.* 1999; Weissenmayer *et al.* 2002) found that it was associated with phosphate limitation. In order to assess whether the ORF32-mediated biosurfactant production is regulated by phosphate in *P. putida,* Basal Media 2 (BM2), a chemically defined minimal media, was selected to establish the influence of phosphate limitation on biosurfactant production.

Media containing < 400 μM phosphate is considered to be phosphate limiting (Mulcahy *et al.* 2010; Bains *et al.* 2012). Below this limit many genes associated with phosphate limitation signalling or uptake such as *phoB* are upregulated, including *olsA* and *olsB* in *P. aeruginosa* (Bains *et al.* 2012), and are indicators of the cell's response to phosphate limitation stress. When *P. putida* MBD1-E54F3 cultures were tested using BM2 media containing 400 μM and 1600 μM phosphate, emulsification activity was only observed under low phosphate conditions, while no emulsion was obtained for the mutant *P. putida* MBD1-E54F3_D1 H5 (Figure 12).

Cultures of *P. putida* MBD1-E54F3 in low phosphate conditions were able to produce the 70% $EC_{24}$ emulsions (Figure 13). However, in prior experiments, *P. putida* MBD1-E43F3 was able to form emulsions with paraffin when cultured in Lysogeny Broth (LB), considered to be a rich medium that is phosphate replete. Unlike *E. coli*, *P. putida* has a putative OlsA, which offers the potential for it to convert any LOL derived from ORF32 expression to OL. There are two scenarios possible in terms of the biosurfactant structures produced. The first, ORF32 expression is phosphate regulated and only produces LOL under low phosphate conditions, which is either complemented by phosphate regulated OlsA resulting in OL production or if OlsA is not involved would only result in LOL accumulation. The second, ORF32 is constitutively expressed and OlsA is expressed under low phosphate conditions. In order to determine which scenario was the most likely, total lipid extractions and TLC analysis was conducted to detect the different forms of ornithine containing lipids and under phosphate control.

*Total lipid extraction*

Ornithine lipids were extracted according to the Bligh and Dyer method (Bligh and Dyer 1959a). *P. putida* culture (1 ml) was extracted by vortex mixing for 20 minutes with 3.75 ml chloroform/methanol (1:2). Following extraction, 1.25 ml chloroform was added and mixed by vortex for 1 minute, a further 1.25 ml $dH_2O$ was mixed by vortex for another minute. The phases were separated by centrifugation at 6 800 x g for 10 minutes. The upper aqueous phase was discarded and the lower organic phase recovered. The chloroform was evaporated and lipid extracted was redissolved in 100 μl chloroform/methanol (2:1). The volumes are proportionally scaled up for larger scale extractions.

*Thin layer chromatography*

Extracts were evaluated for ornithine lipid production using aluminium backed TLC silica gel 60 plates, 20 cm x 20 cm (Merck). The mobile phased used was chloroform/methanol/water (60:25:4) (Weissenmayer *et al.* 2002). Detection of the ornithine lipid was performed by staining with 0.2% ninhydrin in acetone for the amino acid moiety. Ornithine containing bands were scraped off and extracted with chloroform/methanol (2:1) by vortex mixing for 20 minutes. The solvent was dried and dissolved in methanol, which was filtered through a 0.22 μm filter to remove residual silica particles.

In previous studies of ornithine lipid production, the TLC profiles of total lipid extracts from *P. aeruginosa* (Lewenza *et al.* 2011) and *S. meliloti* (Weissenmayer *et* al. 2002) displayed a consistent profile of the phosphatidylethanolamine (PE) under high phosphate conditions and the appearance of ornithine lipid under phosphate poor conditions.

Based on these results the TLC profiles of *P. putida* MBD1-E54F3 and *P. putida* MBD1-E54F3_D1H5 were compared under high (1600 μM) and low (400 μM) phosphate conditions. In the presence of low phosphate, a putative ornithine lipid spot appeared on a ninhydrin stained TLC of total lipid extracts from *P. putida* MBD1-E54F3 but not for *P. putida* MBD1-E54F3_D1H5, nor under high phosphate growth (Figure 14). This represents the first confirmation that OL is produced, and that therefore the endogenous OlsA is involved in the production of the biosurfactant activity observed in *P. putida* MBD1-E54F3 cultures. Given that OlsA expression in other bacteria is known to be phosphate regulated, it is presumed that the phosphate dependent production of OL is a result of the *olsA* regulation. However, this does not necessarily indicate that ORF32 is constitutively expressed, and may also be under phosphate regulation.

*P. putida* MBD1-E54F3_D1H5 produced a weakly stained and inconsistent spot at the position of the putative OL (Figure 15). A similar pattern was observed for the replicate total lipid extractions of *P. putida* MBD1, where a weakly stained and inconsistent spot was observed (Figure 15). This could represent the OL that is produced by *P. putida* via the putative endogenous *olsA* and *olsB* genes, PP0923 and PP0924. To establish why ORF32 results in extra biosurfactant production, despite the presence of endogenous *olsA* and *olsB*-like genes, a time course experiment was conducted. This could confirm the hypothesis that ORF32 produces LOL constitutively, which then serves as a pool of substrate for the *P. putida* MBD1 OlsA (PP0924) to convert LOL to OL.

The time course experiment revealed that *P. putida* MBD1 produced, or at least accumulated, ornithine lipid at a slightly later stage of growth compared to *P. putida* MBD1+E54F3 (OL is faintly visible at OD600nm > 0.6 for MBD1 vs significant production of OL at OD600nm of 0.5 for *P. putida* MBD1+E54F3) (Figure 14). Lyso-ornithine lipid was

never detected in any of the cultures as no band was determined to migrate with synthetic lyso-ornithine lipid synthesised from ornithine and palmitoyl sodium salt (Figure 15). However, the lack of LOL detection might be due to the organic extraction method used to target total lipids (Bligh and Dyer 1959b). Single fatty acid chained amino lipid molecules such as LOL would not be as hydrophobic as the double fatty acid chained OL. Total lipid extraction was also previously conducted for overexpressed *S. meliloti* OlsB in *E. coli,* there too LOL was barely detectable (Gao *et al.* 2004). Other putative biosurfactant long chain N-acyl amino acids (Brady and Clardy 2000), also consisting of a single fatty acid chain coupled to an amino acid, were extracted using ethyl acetate rather than chloroform/methanol (Brady *et al.* 2004). Ethyl acetate extractions might be useful in the future to detect LOL. Nevertheless, the results presented in Figure 13 suggest that the endogenous *P. putida* putative *olsA* is sufficiently expressed to convert the pool of LOL expressed by ORF32 to OL.

It is further likely that LOL is not present in sufficient amounts to be detected, due to ORF32's and OlsA induction by phosphate limitation and the immediate conversion of LOL to OL. If ORF32 was constitutively expressed, then under high phosphate conditions, biosurfactant activity would have been detected in BM2 media due to the accumulation of LOL, which has been shown for the *E. coli* BL21 pET21a-ORF32 experiments to be biosurfactant active. The lack of biosurfactant activity in high phosphate suggests that ORF32 expression is phosphate regulated, and is not constitutively expressed. The apparent contradictory results when *P. putida* MBD1-E54F3 is cultured in LB media might be due to the nutritional deficiencies that occur during late stationary phase (Soberón-Chávez *et al.* 2005), which then would result in phosphate limitation and thus induced expression of ORF32.

*Mass spectrometry*

The TLC extract was confirmed for the presence of ornithine lipids by direct injection mass spectrometry of the TLC extract. The mass spectrometry was conducted with a Perkin Elmer SQ 300 Mass Spectrometer. The fragmentation was effected by ramping of the capillary exit voltage and through collision induced dissociation. Presence of the ornithine lipid was confirmed by comparing the MS speaks to published MS spectra (Geiger *et al.* 1999; Lewenza *et al.* 2011).

Confirmation that the second spot appearing under phosphate limitation in *P. putida* MBD1-E54F3 was an ornithine lipid was conducted by direct injection mass spectrometry of the spot extracted by preparative TLC (Figure 16). The results were compared to the mass spectrometry (MS) profiles of *P. aeruginosa* PA01 previously determined by Lewenza *et al.* Attempts to extract ornithine lipids of the *P. putida* MBD1 from the TLC to perform mass spectrometry were unsuccessful, likely due to the low production of the ornithine lipid.

A positive-ion mode electrospray MS analysis revealed the same signal cluster previously described in *P. aeruginosa* PA01 from 598 to 706 m/z that contained the 115 m/z ion characterising a mixture of major and minor signals as molecular ions of ornithine lipid (Geiger *et al.* 1999; Lewenza *et al.* 2011).

The same signals were observed in the *P. putida* MBD1-E54F3 samples where fragmentation revealed the typical ornithine lipid signals, at 131 m/z representing the ornithine ion, at 115 m/z representing the B-ion of ornithine, and at 70 m/z representing the immonium ion (Geiger *et al.* 1999). These results confirm that the band appearing below the PE band under phosphate limiting conditions is an ornithine lipid.

The major signals, 625.73 and 651.72 observed by Lewenza *et al.* were present but not 665 m/z. The structure of the molecules responsible for these signals were determined by negative ion spray tandem MS by Lewenza *et al.* Each molecule differs in the chain length for the N-linked fatty acid, which is transferred by OlsB. The 665 m/z signal represents an ornithine lipid containing a 19 carbon chain amide linked fatty acid chain and an ester linked 16 carbon fatty acid chain produced by *P. aeruginosa* (Figure 17, third structure), therefore the OlsB of *P. aeruginosa* produces a 19 carbon fatty acid chained lyso-ornithine lipid. The lack of 665 m/z signal suggests that unlike the OlsB of *P. aeruginosa* the ORF32 is only capable of producing the 16 carbon and/or 18 carbon chain, lyso-ornithine lipid (Figure 17).

The most prominent signal was at 651.72 m/z. The equivalent signal was determined by negative spray tandem MS to be a mixture of two molecules of ornithine lipid consisting of two chains of 16 C and 18 C to form an ornithine 34:1 (Figure 18).

Further MS analysis of the total lipid extracts of *P. putida* MBD1-E54F3 is required to properly evaluate the diversity of ornithine containing lipids produced. The presence of two putative *olsB* genes, ORF32 and PP0924 working in concert with a putative *olsA* (PP0923) is expected to result in the production of a mixture of OL: those endogenously produced by *P. putida* MBD1 (PP0924/PP0923) and those produced by ORF32/PP0923. The paraffin spray assay could not detect biosurfactant activity by *P. putida* MBD1, which suggests that the addition of the ORF32 enables biosurfactant production above the limit of the detection assay. ORF32 might either be constitutively expressed or more active than PP0924, which is regulated by phosphate availability. The comparisons of the OL and LOL concentrations between *P. putida* MBD1 and *P. putida* MBD1-E54F3 would quantify the impact of ORF32 on the ornithine containing lipid production of *P. putida* MBD1. Furthermore, comparison of the LOL produced by *E. coli* BL21-ORF32 and *P. putida* MBD1-E54F3 will determine how the different metabolic environments and acyl-ACP availability affects the types of ornithine containing lipids produced. A large scale preparation of *P. putida* MBD1+E54F3 cultures would be necessary to properly ascertain if the presence of OL and LOL is solely responsible for the strong emulsification activity observed. Quantitative analysis

of surface tension by the Du Nouy Ring method and critical micelle concentration is also required to fully characterise the biosurfactant produced here. This requires optimisation of the extraction method to obtain the active fractions purified to an extent where it could be physically characterised or used for applications requiring water-in-oil emulsification.

Calculations of the Hydrophilic Lipophilic Balance (HLB) for ornithine lipid (34:1) in particular obtains a value of 3.45. This value predicts the behaviour of the surfactant to be an anti-foaming agent and a water-in-oil emulsifier. The water-in-oil emulsification has already been observed in the emulsification experiments (Figure 13). Lyso-ornithine lipid (18:1) has an HLB of 6.3 predicting the molecule will have water-in-oil surfactant function. The emulsions observed for both *P. putida* MBD1-E54F3 and *E. coli* BL21-ORF32 are creaming emulsions, where the buoyant droplets emulsion tend to rise to the top of container. Creaming emulsions are of high commercial importance where the major concern in a process food emulsion is the onset of visual non-uniformity (Robins 2000).

*Organic synthesis of LOL by the N-acyl amino acid synthetic method*

The chemical synthesis of lyso-ornithine lipid was conducted according to the N-acyl amino acid synthetic method (Molineroa *et al.* 1988). An aqueous solution of the sodium salt of L-ornithine was prepared by increasing the pH to 10 using NaOH, the solution was chilled to 0°C. Mysteroyl chloride was added dropwise to the cooled aqueous solution under moderate stirring. After addition of the ornithine, the solution was stirred at room temperature for 5 hours at room temperature. N-acyl L-ornithine was purified from the reaction mixture by silica gel column chromatography using a step-wise gradient of chloroform/methanol from 100% chloroform to 0%.

**EXAMPLE 9**

In vitro *transposon mutagenesis of E54F3*

*In vitro* mutagenesis was conducted on the E54F3 fosmid to confirm that ORF32 was responsible for the activity. The EZ-Tn5 <TET> insertion kit was using to randomly insert a transposon into the fosmid and provides tetracycline resistance to mutant clones for selection. The reaction was performed using 200 ng of fosmid DNA extracted using the Qiagen Midiprep Kit, which for 41 kb E54F3 is 0.0074 pmols. A molar equivalent amount of EZ-Tn5 Transposon was added to the reaction, with 1 µl Transposases, 1 µl EZ-Tn5 reaction buffer and $dH_2O$ to a final volume of 10 µl. The reaction was incubated at 37°C for 2 hours. After incubation 1 µl 10 x stop solution and heated for 10 minutes at 70°C. The reaction was either stored at -20°C or 1 µl was used to electroporate competent *E. coli* Epi300 containing the helper plasmid then conjugated into *P. putida* MBD1 to test for lack of biosurfactant activity by the paraffin droplet spray method. Biosurfactant negative clones were selected and the *olsB* amplified using the olsB873 primers. Insertion into the *olsB* ORF32 was detected by a 1.2 kb shift in amplicon size. The insertion was mapped using the transposon specific primers FP1 and RP1.

**EXAMPLE 10**

*Bacterial RNA extraction*

Total RNA was extracted from *P. putida* MBD1, *P. putida* E43F3 and *E. coli* E54F3 cultured in both LB broth and BM2 media supplemented with high and low phosphate concentrations. The expression of the *olsB* gene was determined by amplification of olsB873 amplicon.

*RNA preparation*

Total bacterial RNA was isolated by TRIzol™ reagent (ThermoFisher Scientific) method. The *P. putida* E54F3 and MBD1 were grown in LB or BM2 to mid log phase. Culture samples (1 ml) were harvested by centrifugation at top speed in a benchtop centrifuge at 4°C for 5 minutes. The culture supernatant was discarded. After, centrifugation the cell pellets were suspended in 1 ml TRIzol™ reagent, which was incubated at room temperature for 5 minutes. A volume of 200 µl per 1 ml TRIzol™ chloroform was added to the mixture and shook by hand for 15 seconds. The sample was incubated at room temperature for 3 minutes and the phases were separated by centrifugation at 12 000 x g at 4°C for 15 minutes. The top aqueous phase was aspirated and transferred to a fresh 1.5 ml microcentrifuge tube containing 500 µl of 100% isopropanol. The sample was mixed and incubated at room temperature for 10 minutes then centrifuged at 12 000 x g at 4°C for 10 minutes. The isopropanol was carefully discarded and 1 ml 75% ethanol was added to the RNA pellet, which was vortexed briefly. The RNA was harvested by centrifugation at 7500 x g at 4°C for 5 minutes. The ethanol was removed and dried. The RNA was dissolved in 50 µl RNase free molecular grade water.

*DNase treatment*

The RNA preparations were treated with DNase I (New England Biolabs) where 30 μl of RNA extract was added to 10 μl 10 x DNase I reaction buffer, 60 μl of RNase free $dH_2O$ and 1 μl (2U) DNase I. The reaction mixture was incubated at 37°C for 10 minutes followed by the addition of 1 μl 500 mM EDTA. The reaction mixture was inactivated by incubation in a 75°C water bath for 15 minutes. The effectiveness of the DNase I treatment was evaluated by PCR amplification of 16S rDNA sequence. Absence of product indicates all DNA was removed from the RNA sample.

*Evaluation of RNA quality*

The RNA was examined for quality by bleach agarose gel electrophoresis (Aranda *et al.* 2012). Sodium hypochlorite (6%) was added to a final volume of 1% (v/v) to a 1% agarose mixture. The agarose was incubated at room temperature for 10 minutes before melting and pouring into the gel casting apparatus.

*Complementary DNA synthesis (cDNA)*

cDNA synthesis was performed on DNase I treated RNA using the First Strand cDNA Synthesis Kit for RT-PCR AMV (Roche) according to the manufacturers recommendations. Each reaction 1 μg of total RNA was used and synthesis was conducted using random primers. Synthesis was confirmed by 16S rDNA sequence amplification.

## Claims

1. Use of a recombinant microorganism capable of heterologously expressing an ornithine acyl-ACP N-acyltransferase which catalyses the synthesis of lyso-ornithine lipid, and wherein the ornithine acyl-ACP N-acyltransferase is heterologously expressed by an *olsB*-like gene having at least 90% sequence identity with SEQ ID NO:1, as a biosurfactant.

2. The use of claim 1, wherein the recombinant microorganism is selected from recombinant *Pseudomonas putida* or *Escherichia coli.*

3. The use of claim 1 or claim 2, wherein the recombinant microorganism endogenously expresses lyso-ornithine dependent acyl-ACP O-acyltransferase.

**4.** The use of claim 3, wherein the lyso-ornithine dependent acyl-ACP O-acyltransferase is expressed by an *olsA*-gene present in the microorganism.

**5.** The use of claim 3 or claim 4, wherein the lyso-ornithine dependent acyl-ACP O-acyltransferase catalyses the synthesis of ornithine lipid.

**6.** The use of any one of claims 1 to 5, wherein the biosurfactant has an emulsification capacity after 24 hours ($EC_{24}$) of at least 65% when emulsifying in paraffin.

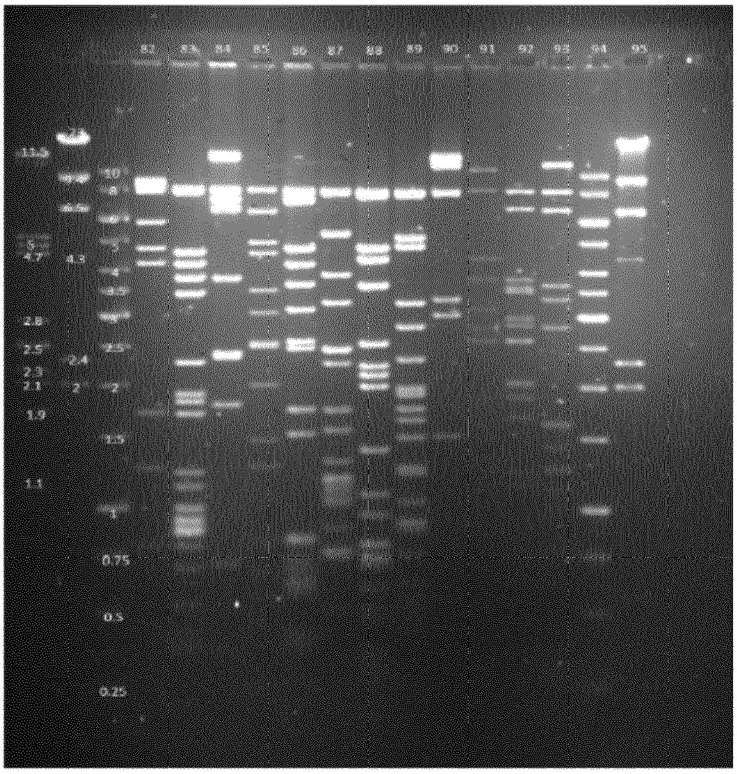

Figure 1

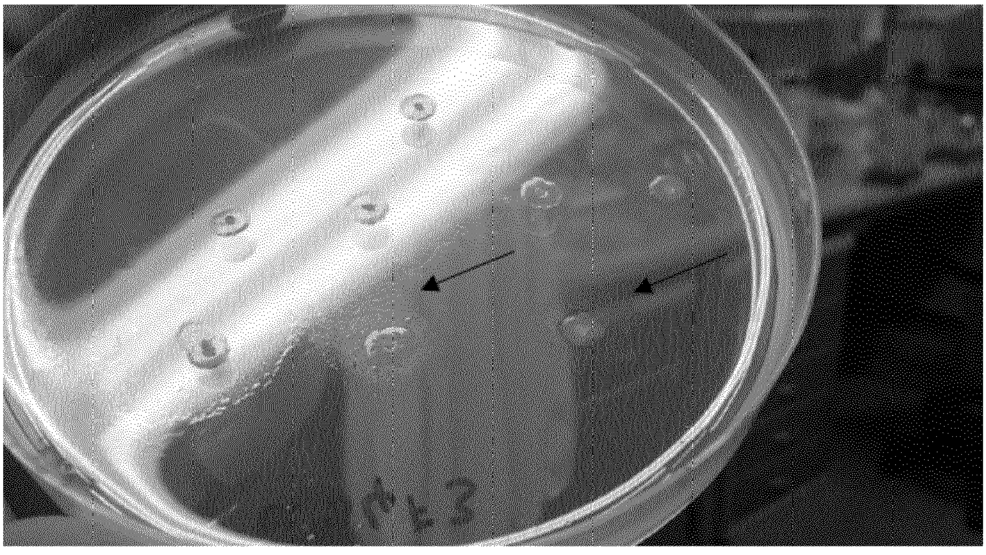

Figure 2

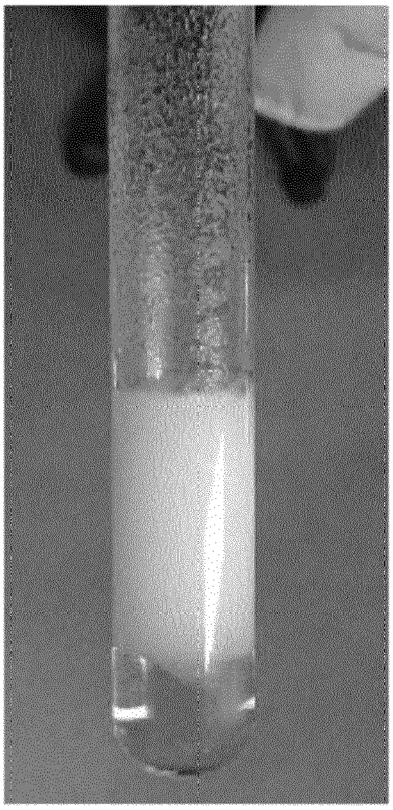

Figure 3

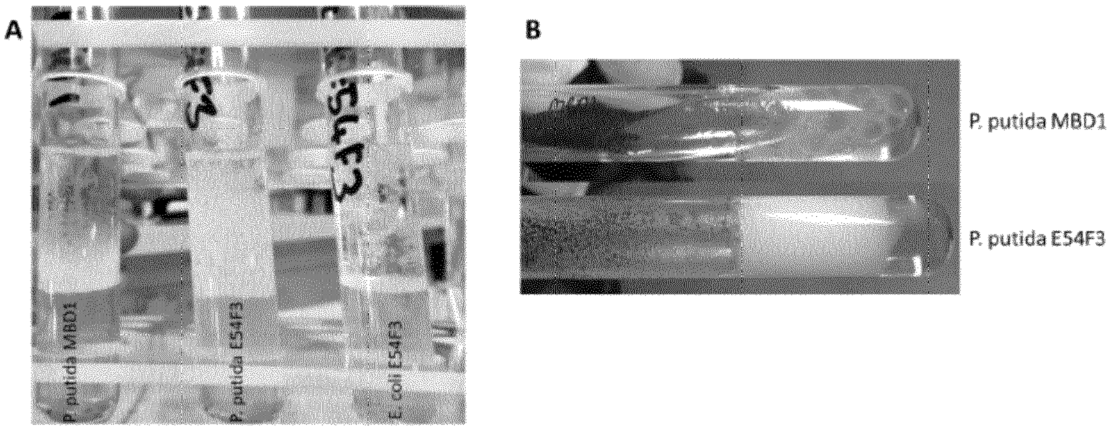


Figure 4

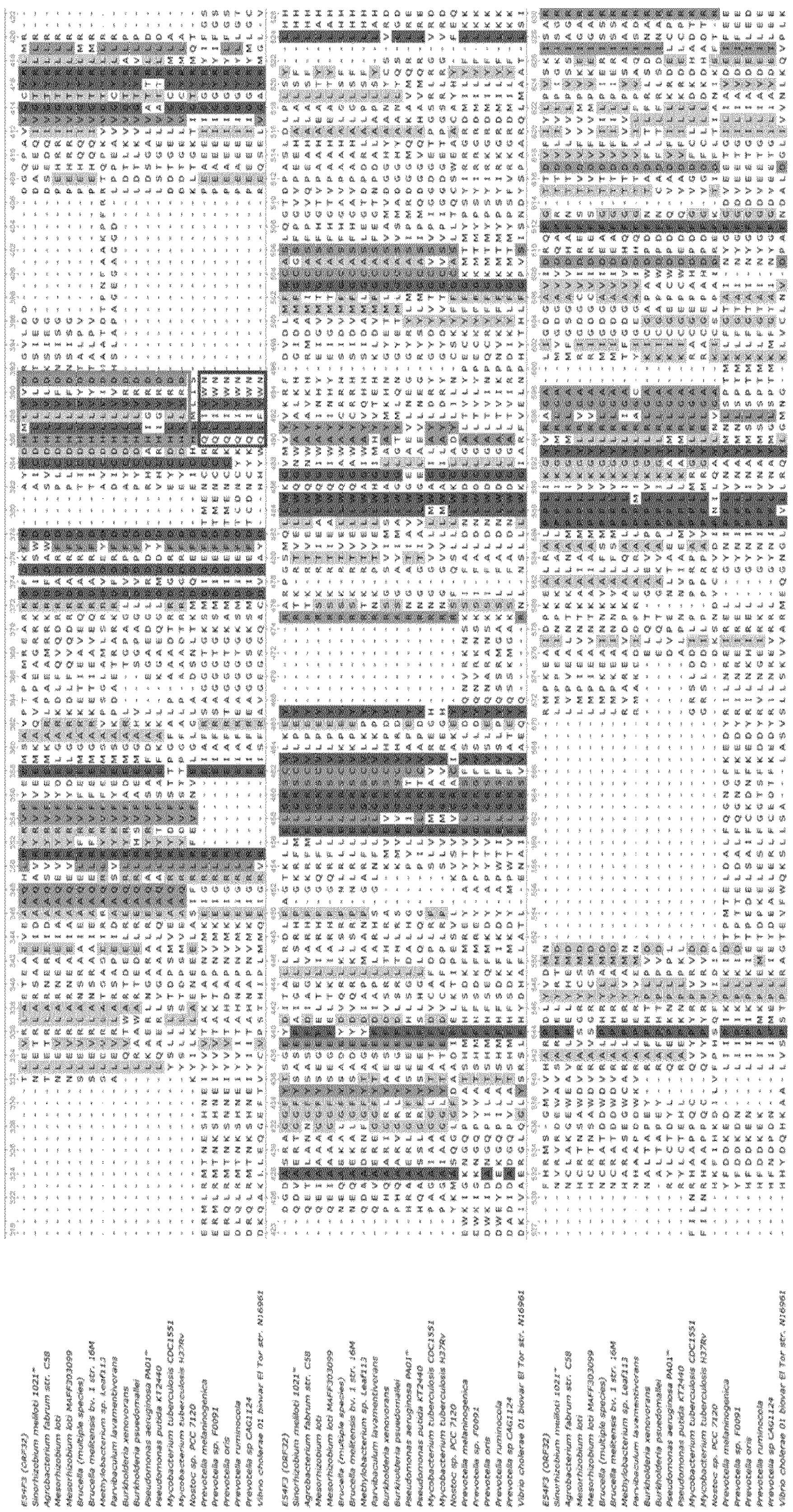

Figure 5

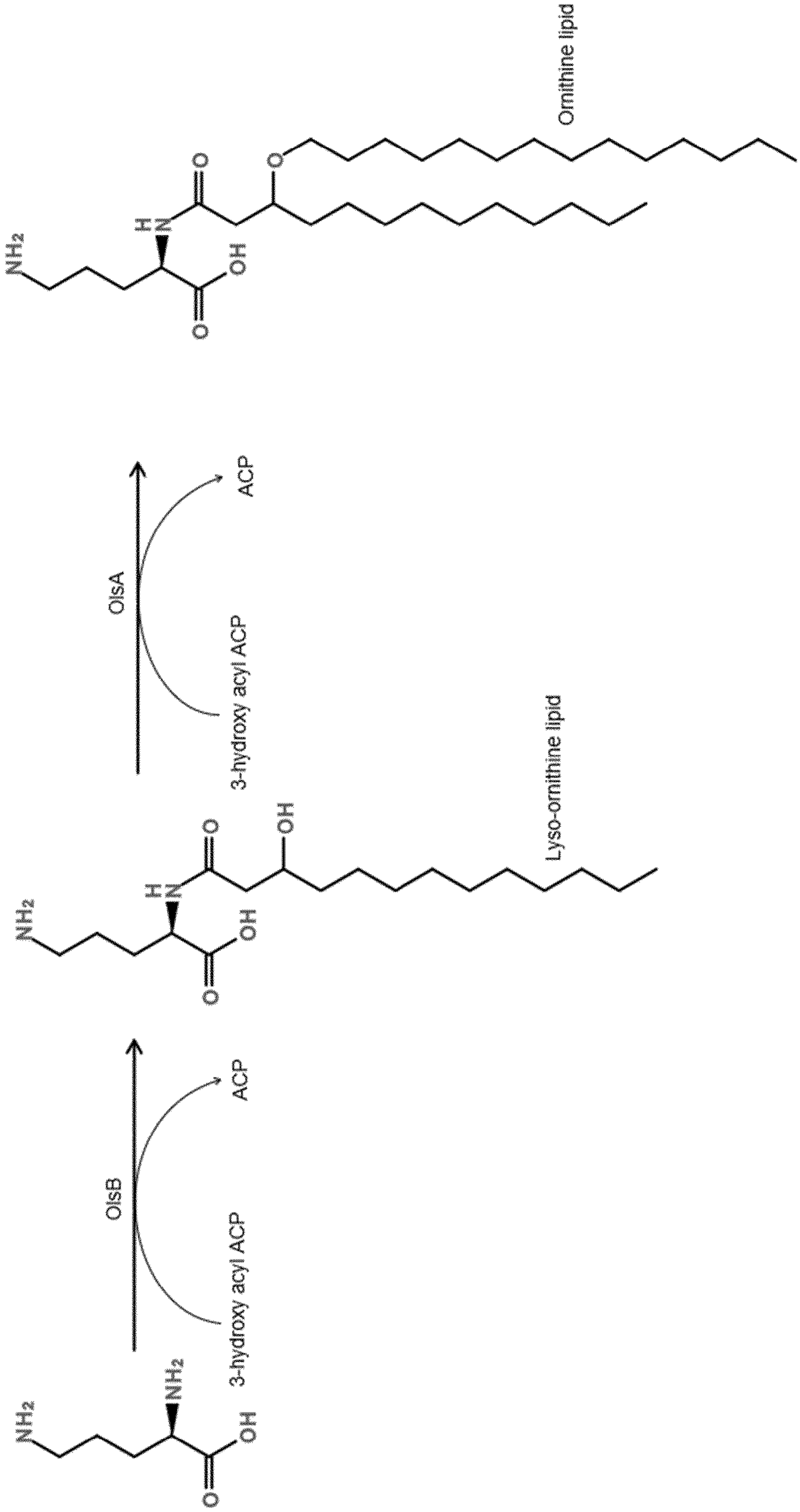
OlsB
3-hydroxy acyl ACP
ACP
Lyso-ornithine lipid
OlsA
3-hydroxy acyl ACP
ACP
Ornithine lipid

Figure 6

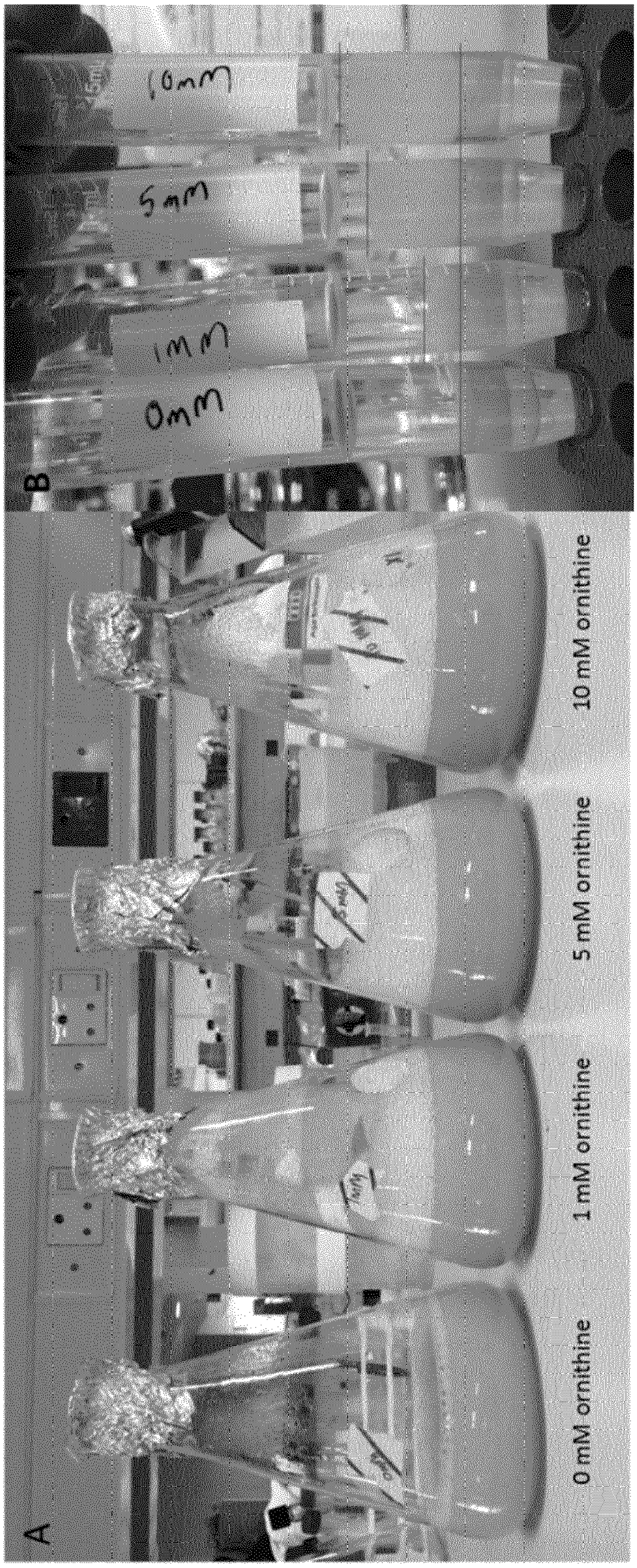
A
0 mM ornithine
1 mM ornithine
5 mM ornithine
10 mM ornithine
B
0mM
1mM
5mM
10mM

Figure 7

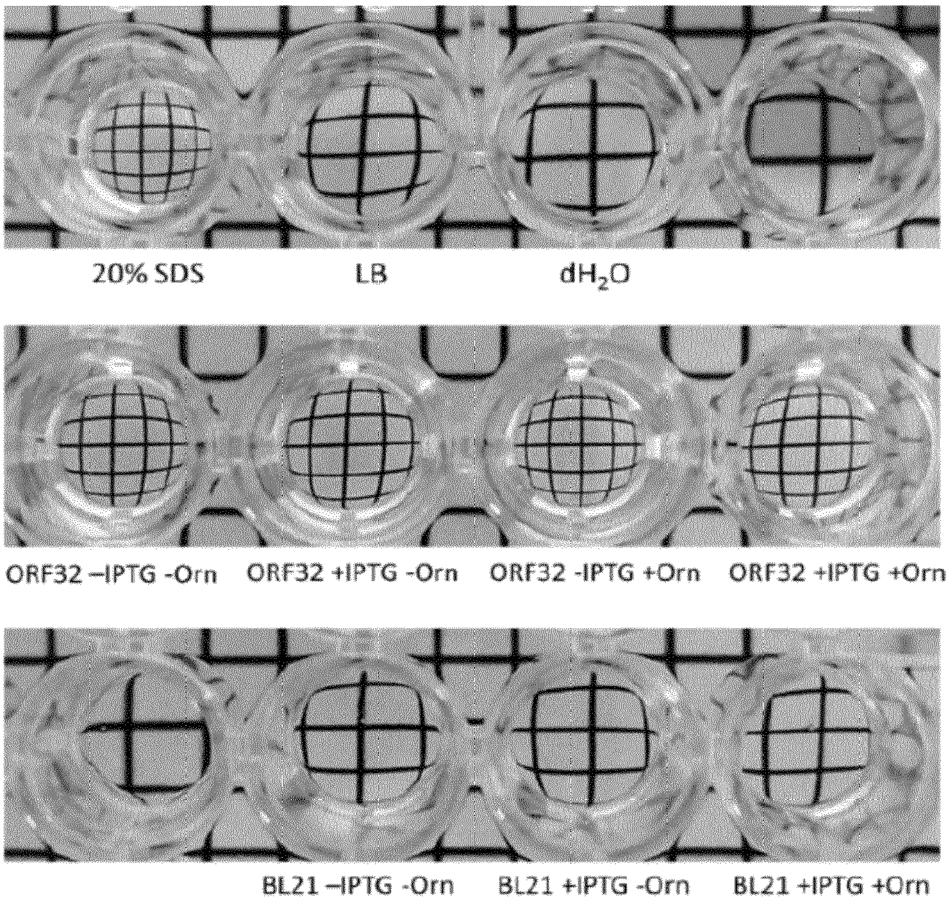
20% SDS
LB
$dH_2O$
ORF32 –IPTG -Orn
ORF32 +IPTG -Orn
ORF32 -IPTG +Orn
ORF32 +IPTG +Orn
BL21 –IPTG -Orn
BL21 +IPTG -Orn
BL21 +IPTG +Orn

Figure 8

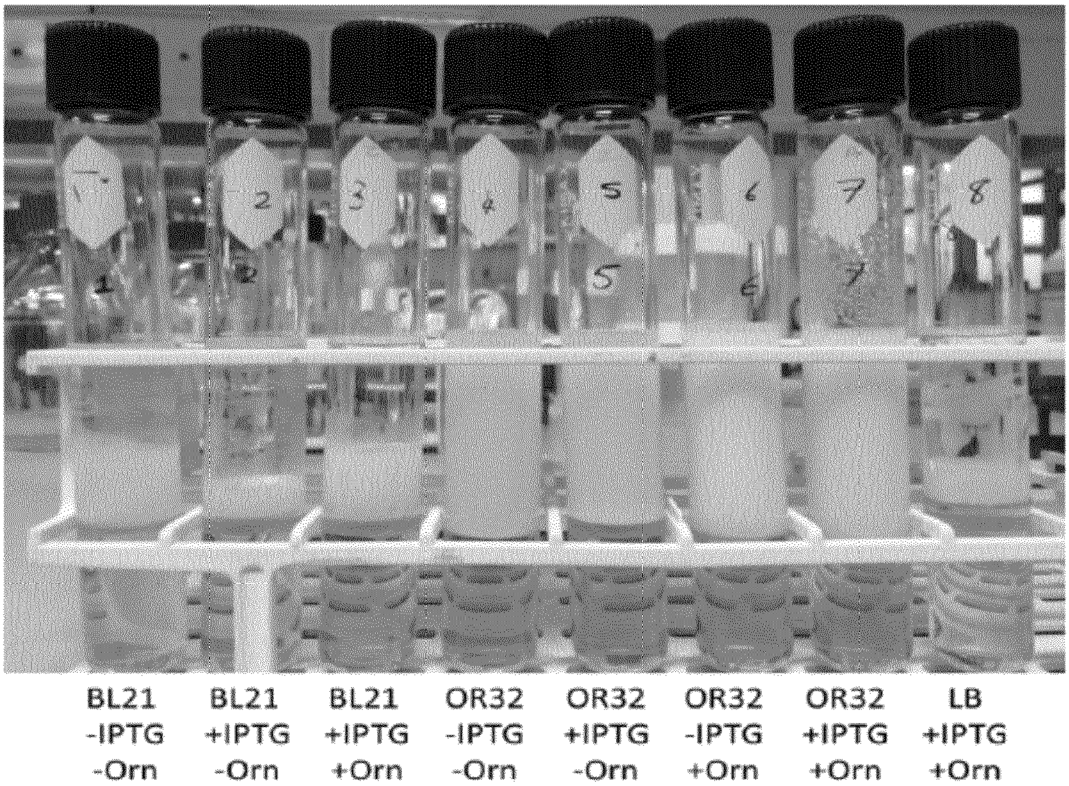


Figure 9

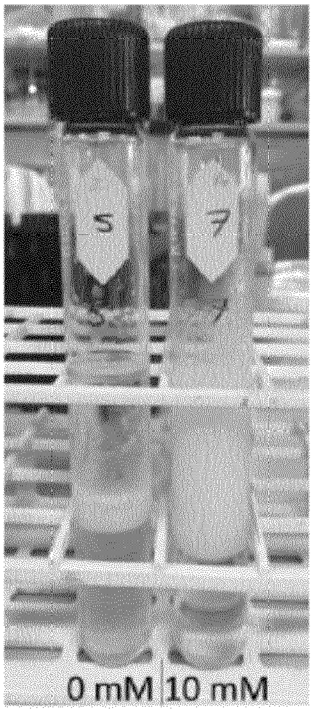


Figure 10

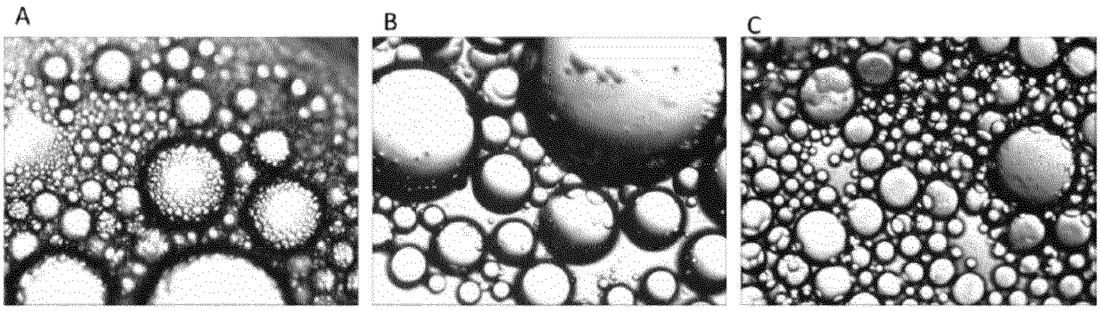


Figure 11

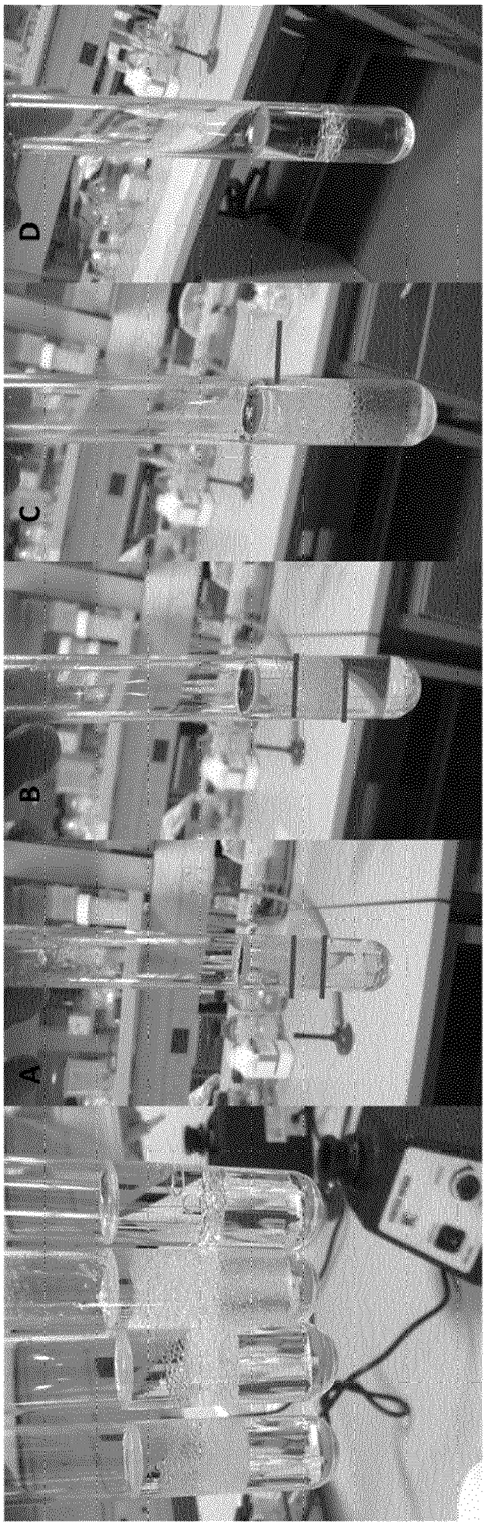


P. putida E54F3
High $PO_4$

P. putida E54F3_D1E5
High $PO_4$

P. putida E54F3
Low $PO_4$

P. putida E54F3_D1E5
Low $PO_4$

Figure 12

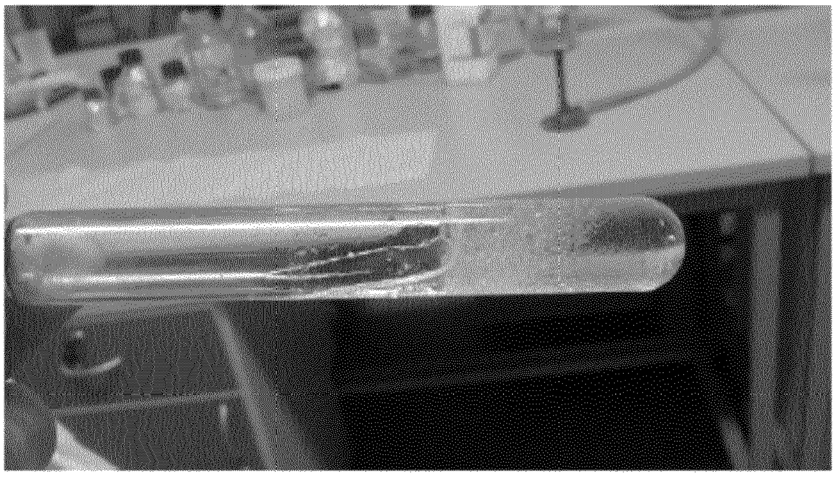

Figure 13

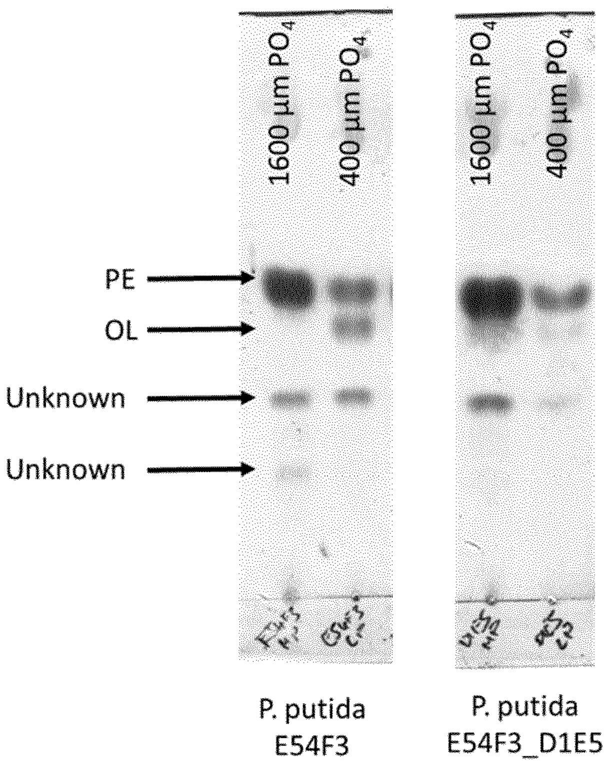


Figure 14

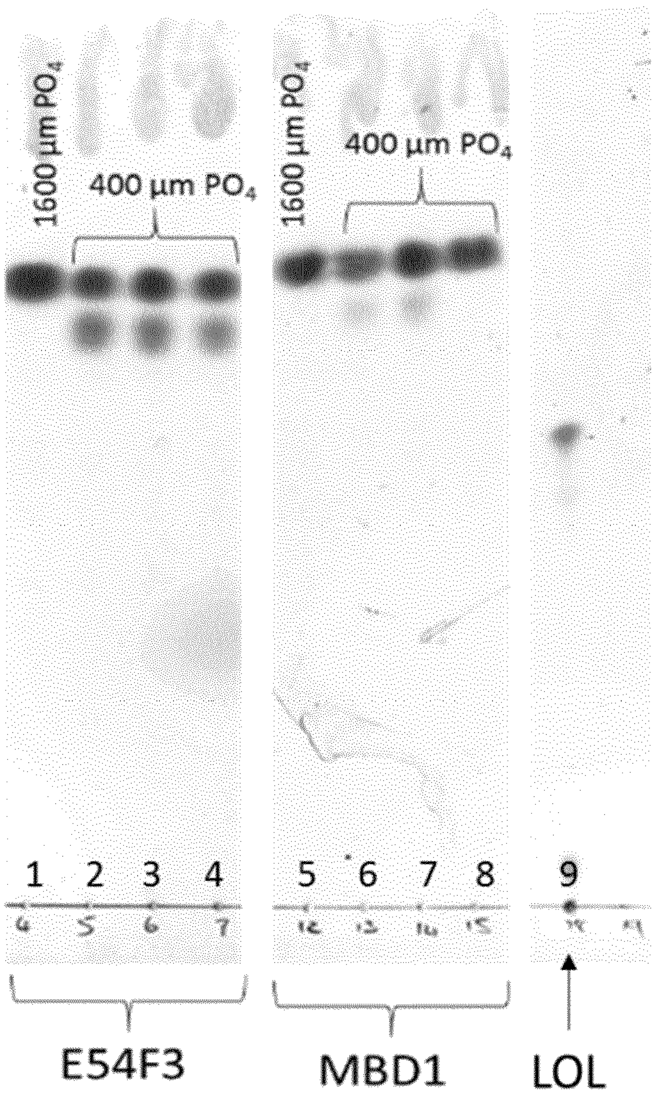
1600 µm $PO_4$
400 µm $PO_4$
1600 µm $PO_4$
400 µm $PO_4$
1 2 3 4
5 6 7 8
9
E54F3
MBD1
LOL

Figure 15

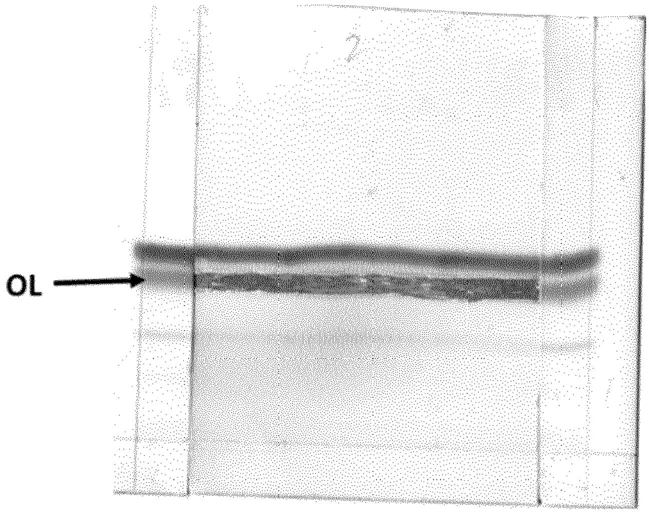


Figure 16

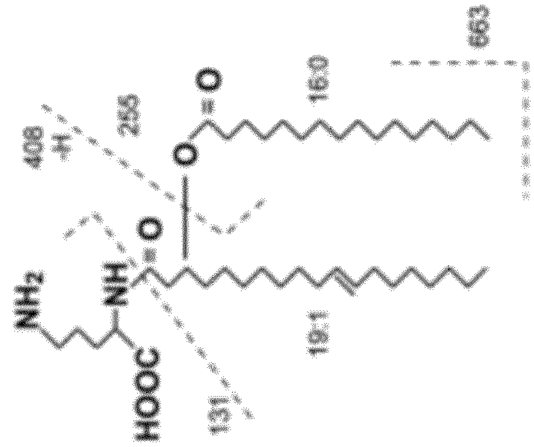
408
-H
255
16:0
663
19:1
131

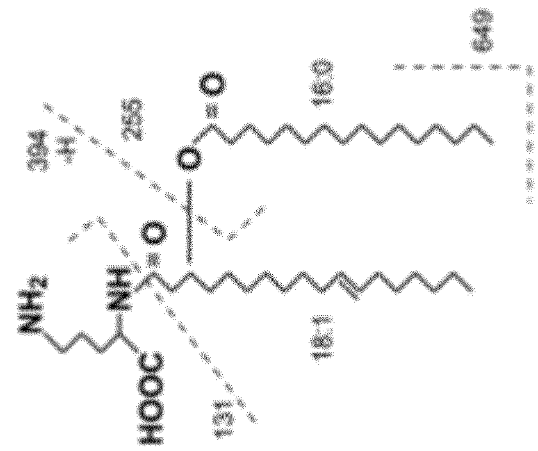
394
-H
255
16:0
649
18:1
131

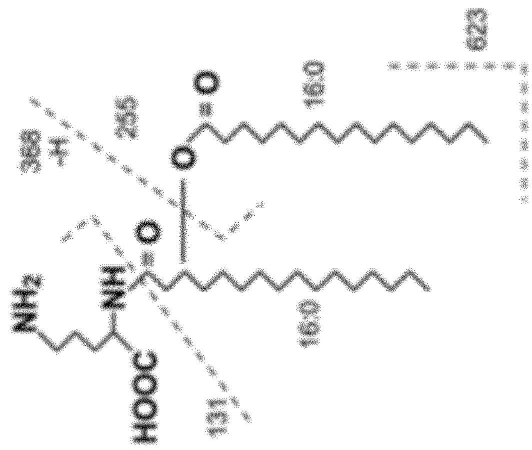
368
-H
255
16:0
623
16:0
131

Figure 17

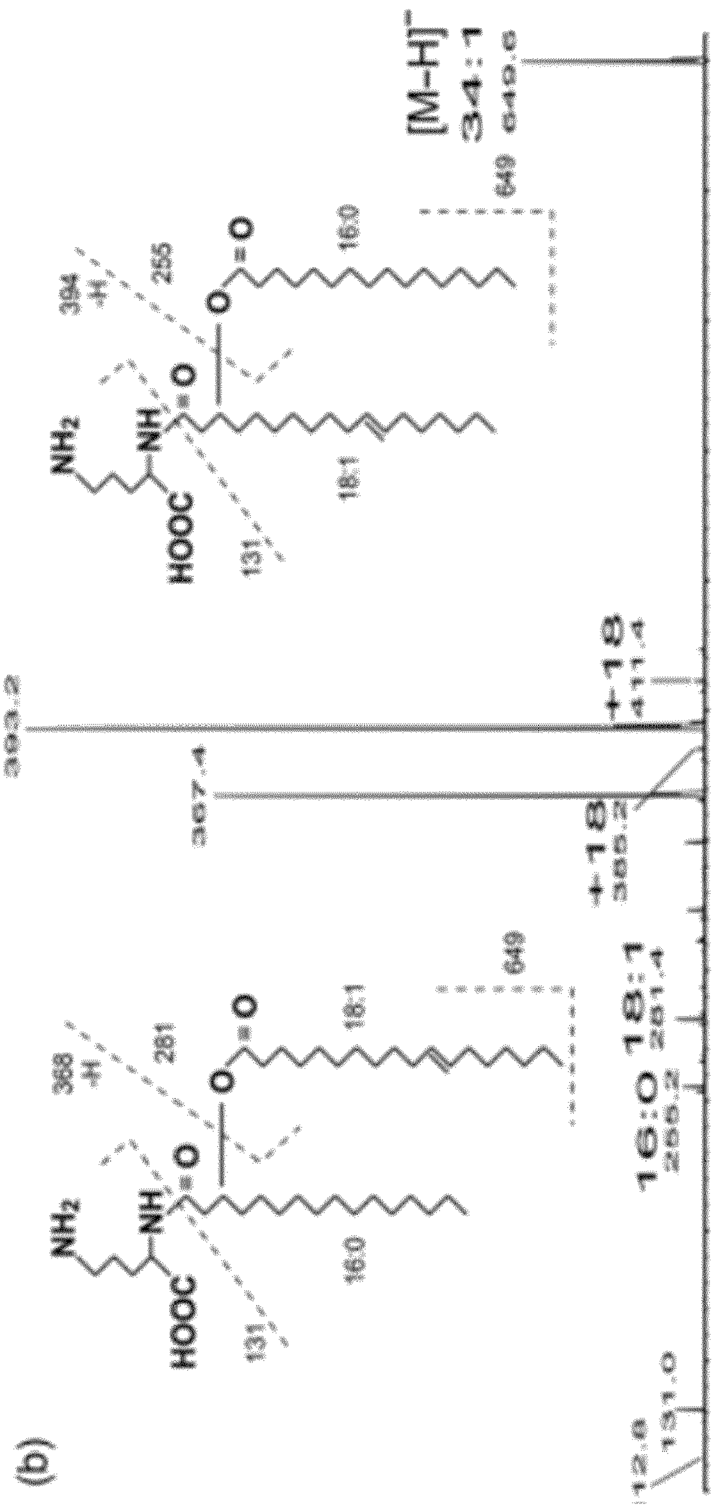
(b)
[M-H]-
34:1
649.6
393.2
367.4
+18
411.4
+18
385.2
16:0
255.2
18:1
281.4
131.0
412.8

Figure 18

TTGGTAAATATTTACGAGCCTGGTCCGACCGATGGACGTCTCGAGCAGT
GGCCGATTCTTGCGACATCGGGAACGCTCGAAGTGCGGCTGGCCGAAAC
CGAAGCCGAGGTCGAAGCCGCCCAGCATCTTCGCTATCGCGTTTTTTAT
GAAGAGATGTCTGCCGTGCCGACACCGGCCATGCGCGAGGCCCGGCGCG
ACTTCGACAAGTTCGATGCCTATTGCGATCACATGCTGGTGGTCGACCG
CGGTGTGCTCGACGATGATGGACAACCTGCCGTCGTCGGCTGCTACCGC
CTGATGCGCGACGGCGATGCCTCCCGCGCCGGCGGCTTTTACACCAGCG
GCGAATACGACATCACGGCTTTGCTTCGCGATCTCCCCGCCGGAACCAA
GCTTCTCGAACTCGGCCGCTCCTGCGTCTTGAAGGAATATCGCGCGAGG
CCCGGGAGCATGCAGCTCCTGTGGAAAGGCGTGATGGTTTACGTCAAAC
GTTTCGACGTCGATCTGATGTTCGGCTGCGCCTCGCTGCAGGGGACCGA
TCCTGCCTCGCTCGACCTGCCGCTCTCATACCTGCATCATTTTCATCGC
ATGAGCGGAATGGTCGTTCACGCCAGACCCGATCTCTATGTCGATATGA
ACCGGATGCCGAAAGAAGCCATCGATCCGAAGGAAGCGCTTAGGGCGTT
GCCGCCTCTCATCAAGGGTTATGTCCGTGCCGGGGCCCTGATCGGCGAC
GGCGCGGTGATCGACCGGCAATTCGGCACCACCGACGTCTTCATCTACC
TGCCGGTTTCCAAGATCAGCGCGCGCTACCAGAGCCGTTTCGGCCAAAG
CCGCTGA

Figure 19

LVNIYEPGPTDGRLEQWPILATSGTLEVRLAETEAEVEAAQHLRYRVFY
EEMSAVPTPAMREARRDFDKFDAYCDHMLVVDRGVLDDDGQPAVVGCYR
LMRDGDASRAGGFYTSGEYDITALLRDLPAGTKLLELGRSCVLKEYRAR
PGSMQLLWKGVMVYVKRFDVDLMFGCASLQGTDPASLDLPLSYLHHFHR
MSGMVVHARPDLYVDMNRMPKEAIDPKEALRALPPLIKGYVRAGALIGD
GAVIDRQFGTTDVFIYLPVSKISARYQSRFGQSR

Figure 20

Europäisches Patentamt
European Patent Office
Office européen des brevets

# EUROPEAN SEARCH REPORT

Application Number
EP 20 16 8557

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MANEERAT ET AL: "Characterization of cell-associated bioemulsifier from Myroides sp. SM1, a marine bacterium", SONGKLANAKARIN JOURNAL OF SCIENCE AND TECHNOLOGY, vol. 29, 2007, pages 769-779, XP002776031, * See pages 770-771 (Introduction/ ornithine lipids) * * page 773, left-hand column, paragraph 2 - right-hand column, paragraph 1; table 1 * | 1-6 | INV. C12N9/10 C12P13/10 C12P7/64 C11D1/52 C11D1/64 C12P13/04 |
| A | SIEBERS ET AL: "Lipids in plant-microbe interactions", BIOCHIMICA ET BIOPHYSICA ACTA, vol. 1861, 27 February 2016 (2016-02-27), pages 1379-1395, XP002776028, * See pages 1390-1391 (section 4.2/OLs); Figure 8 * | 1-6 | |
| A | LIDBURY ET AL: "Comparative genomic, proteomic and exoproteomic analyses of three Pseudomonas strains reveals novel insights into the phosphorus scavenging capabilities of soil bacteria", ENVIRONMENTAL MICROBIOLOGY, vol. 18, 7 July 2016 (2016-07-07), pages 3535-3549, XP002776029, * See page 3540 (Table 1/OlsA,OlsB); early online publication * | 1-6 | TECHNICAL FIELDS SEARCHED (IPC) C12P C12N C11D |
| A | AKTAS ET AL: "Membrane lipids in Agrobacterium tumefaciens: biosynthetic pathways and importance for pathogenesis", FRONTIERS IN PLANT SCIENCE, vol. 5, 2014, pages 1-13, XP002776030, * See end of page 5 to page 7 (ornithine lipids) * | 1-6 | |
| | -/-- | | |

The present search report has been drawn up for all claims

2

| Place of search | Date of completion of the search | Examiner |
|---|---|---|
| Berlin | 31 July 2020 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 16 8557

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GRANAFEI ET AL: "Profiling of ornithine lipids in bacterial extracts of Rhodobacter sphaeroides by reversed-phase liquid chromatography with electrospray ionization and multistage mass spectrometry (RPLC-ESI-MSn)", ANALYTICA CHIMICA ACTA, vol. 903, 27 November 2015 (2015-11-27), pages 110-120, XP029364353, * See pages 110-111 (Abstract and Introduction) * ----- | 1-6 | |
| | | | TECHNICAL FIELDS SEARCHED (IPC) |

2

The present search report has been drawn up for all claims

| Place of search | Date of completion of the search | Examiner |
|---|---|---|
| Berlin | 31 July 2020 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

SEQUENCE LISTING

<110> University of the Western Cape

<120> Heterologous Expression of Acyl Transferase Enzymes

<130> P36214EPND1/SNB

<140> PCT/IB2017/055761
<141> 2017-09-22

<150> GB1616230.7
<151> 2016-09-23

<160> 10

<170> PatentIn version 3.5

<210> 1
<211> 840
<212> DNA
<213> Unknown

<220>
<223> Metagenomic sample

<400> 1
ttggtaaata tttacgagcc tggtccgacc gatggacgtc tcgagcagtg gccgattctt 60

gcgacatcgg gaacgctcga agtgcggctg gccgaaaccg aagccgaggt cgaagccgcc 120

cagcatcttc gctatcgcgt tttttatgaa gagatgtctg ccgtgccgac accggccatg 180

cgcgaggccc ggcgcgactt cgacaagttc gatgcctatt gcgatcacat gctggtggtc 240

gaccgcggtg tgctcgacga tgatggacaa cctgccgtcg tcggctgcta ccgcctgatg 300

cgcgacggcg atgcctcccg cgccggcggc ttttacacca gcggcgaata cgacatcacg 360

gctttgcttc gcgatctccc cgccggaacc aagcttctcg aactcggccg ctcctgcgtc 420

ttgaaggaat atcgcgcgag gcccgggagc atgcagctcc tgtggaaagg cgtgatggtt 480

tacgtcaaac gtttcgacgt cgatctgatg ttcggctgcg cctcgctgca ggggaccgat 540

cctgcctcgc tcgacctgcc gctctcatac ctgcatcatt ttcatcgcat gagcggaatg 600

gtcgttcacg ccagacccga tctctatgtc gatatgaacc ggatgccgaa agaagccatc 660

gatccgaagg aagcgcttag ggcgttgccg cctctcatca agggttatgt ccgtgccggg 720

gccctgatcg gcgacggcgc ggtgatcgac cggcaattcg gcaccaccga cgtcttcatc 780

tacctgccgg tttccaagat cagcgcgcgc taccagagcc gtttcggcca aagccgctga 840

<210> 2
<211> 279
<212> PRT
<213> Unknown

<220>
<223> Metagenomic sample

<400> 2

Leu Val Asn Ile Tyr Glu Pro Gly Pro Thr Asp Gly Arg Leu Glu Gln
1               5                   10                  15

Trp Pro Ile Leu Ala Thr Ser Gly Thr Leu Glu Val Arg Leu Ala Glu
            20                  25                  30

Thr Glu Ala Glu Val Glu Ala Ala Gln His Leu Arg Tyr Arg Val Phe
        35                  40                  45

Tyr Glu Glu Met Ser Ala Val Pro Thr Pro Ala Met Arg Glu Ala Arg
    50                  55                  60

Arg Asp Phe Asp Lys Phe Asp Ala Tyr Cys Asp His Met Leu Val Val
65                  70                  75                  80

Asp Arg Gly Val Leu Asp Asp Asp Gly Gln Pro Ala Val Val Gly Cys
                85                  90                  95

Tyr Arg Leu Met Arg Asp Gly Asp Ala Ser Arg Ala Gly Gly Phe Tyr
            100                 105                 110

Thr Ser Gly Glu Tyr Asp Ile Thr Ala Leu Leu Arg Asp Leu Pro Ala
        115                 120                 125

Gly Thr Lys Leu Leu Glu Leu Gly Arg Ser Cys Val Leu Lys Glu Tyr
    130                 135                 140

Arg Ala Arg Pro Gly Ser Met Gln Leu Leu Trp Lys Gly Val Met Val
145                 150                 155                 160

Tyr Val Lys Arg Phe Asp Val Asp Leu Met Phe Gly Cys Ala Ser Leu
                165                 170                 175

Gln Gly Thr Asp Pro Ala Ser Leu Asp Leu Pro Leu Ser Tyr Leu His
            180                 185                 190

His Phe His Arg Met Ser Gly Met Val Val His Ala Arg Pro Asp Leu
        195                 200                 205

Tyr Val Asp Met Asn Arg Met Pro Lys Glu Ala Ile Asp Pro Lys Glu
    210                 215                 220

Ala Leu Arg Ala Leu Pro Pro Leu Ile Lys Gly Tyr Val Arg Ala Gly
225                 230                 235                 240

Ala Leu Ile Gly Asp Gly Ala Val Ile Asp Arg Gln Phe Gly Thr Thr
245 250 255

Asp Val Phe Ile Tyr Leu Pro Val Ser Lys Ile Ser Ala Arg Tyr Gln
260 265 270

Ser Arg Phe Gly Gln Ser Arg
275

<210> 3
<211> 19
<212> DNA
<213> Artificial

<220>
<223> E9F primer - Universal bacterial 16S rRNA

<400> 3
gagtttgatc ctggctcag 19

<210> 4
<211> 19
<212> DNA
<213> Artificial

<220>
<223> U1510R primer - Universal bacterial 16S rRNA

<400> 4
ggttaccttg ttacgactt 19

<210> 5
<211> 21
<212> DNA
<213> Artificial

<220>
<223> OlsB873F primer - Amplifies the E54F3 ORF32

<400> 5
gtgggcgaac agaggaaaaa g 21

<210> 6
<211> 20
<212> DNA
<213> Artificial

<220>
<223> OlsB873R primer - Amplifies the E54F3 ORF32

<400> 6
tttttgtcag cggctttggc 20

<210> 7
<211> 20
<212> DNA

<213> Artificial

<220>
<223> OlsB1029F primer - Amplifies E54F3 olsB ORF plus upstream region

<400> 7
cgaatgccat ccccacatca 20

<210> 8
<211> 20
<212> DNA
<213> Artificial

<220>
<223> OlsB1029R primer - Amplifies E54F3 olsB ORF plus upstream region

<400> 8
gctttttgtc ggcggtcttt 20

<210> 9
<211> 33
<212> DNA
<213> Artificial

<220>
<223> ORF32_NdeI primer - Amplifies E54F3 ORF32 with NdeI restriction site

<400> 9
ggaattccat atgttggtaa atatttacga gcc 33

<210> 10
<211> 36
<212> DNA
<213> Artificial

<220>
<223> ORF32_NotI primer - Amplifies E54F3 ORF32 with NotI restriction site

<400> 10
ataagaatgc ggccgcgcgg ctttggccga aacggc 36